# EUROPEAN PATENT APPLICATION

(11) **EP 1 166 798 A1**
(43) Date of publication of application: **02.01.2002**
(21) Application number: 00250194.8
(22) Date of filing: 23.06.2000
(51) Int. Cl.: A61K 45/06

(54) **Combinations and compositions which interfere with VEGF/VEGF and angiopoietin/ Tie receptor function and their use**

(71) Applicant: Schering Aktiengesellschaft, 13353 Berlin (DE)
(72) Inventor: Siemeister, Gerhard, 13503 Berlin (DE); Haberey, Martin, Dr., 12169 Berlin (DE); Thierauch, Karl-Heinz, Dr., 14169 Berlin (DE)

(57) **Abstract**

The present invention describes the combination of substances interfering with the biological activity of Vascular Endothelial Growth Factor (VEGF)NEGF receptor systems (compound I) and substances interfering with the biological function of Angiopoietin/Tie receptor systems (compound II) for inhibition of vascularization and for cancer treatment.

## Description

The present invention provides the combination of substances interfering with the biological activity of Vascular Endothelial Growth Factor (VEGF)/VEGF receptor systems (compound I) and substances interfering with the biological function of Angiopoietin/Tie receptor systems (compound II) for inhibition of vascularization and for cancer treatment.

Protein ligands and receptor tyrosine kinases that specifically regulate endothelial cell function are substantially involved in physiological as well as in disease-related angiogenesis. These ligand/receptor systems include the Vascular Endothelial Growth Factor (VEGF) and the Angiopoietin (Ang) families, and their receptors, the VEGF receptor family and the tyrosine kinase with immunoglobulin-like and epidermal growth factor homology domains (Tie) family. The members of the two families of receptor tyrosine kinases are expressed primarily on endothelial cells. The VEGF receptor family includes Flt1 (VEGF-R1), Flk1/KDR (VEGF-R2), and Flt4 (VEGF-R3). These receptors are recognized by members of the VEGF-related growth factors in that the ligands of Flt1 are VEGF and placenta growth factor (PIGF), whereas Flk1/KDR binds VEGF, VEGF-C and VEGF-D, and the ligands of Flt4 are VEGF-C and VEGF-D (Nicosia, Am. J. Pathol. 153, 11-16, 1998). The second family of endothelial cell specific receptor tyrosine kinases is represented by Tie1 and Tie2 (also kown as Tek). Whereas Tie1 remains an orphan receptor, three secreted glycoprotein ligands of Tie2, Ang1, Ang2, and Ang3/Ang4 have been discovered (Davis et al., Cell 87, 1161-1169, 1996; Maisonpierre et al., Science 277, 55-60, 1997; Valenzuela et al, Proc. Natl. Acad. Sci. USA 96, 1904-1909, 1999; patents: US 5,521,073; US 5,650,490; US 5,814,464).

The pivotal role of VEGF and of its receptors during vascular development was exemplified in studies on targeted gene inactivation. Even the heterozygous disruption of the VEGF gene resulted in fatal deficiencies in vascularization (Carmeliet et al., Nature 380, 435-439, 1996; Ferrara et al., Nature 380, 439-442, 1996). Mice carrying homozygous disruptions in either Flt1 or FIk1/KDR gene die in mid-gestation of acute vascular defects. However, the phenotypes are distinct in that Flk1/KDR knock-out mice lack both endothelial cells and a developing hematopoietic system (Shalaby et al. Nature 376, 62-66, 1995), whereas Flt1 deficient mice have normal hematopoietic progenitors and endothelial cells, which fail to assemble into functional vessels (Fong et al., 376, 66-70, 1995). Disruption of the Flt4 gene, whose extensive embryonic expression becomes restricted to lymphatic vessels in adults, revealed an essential role of Flt4 for the remodeling and maturation of the primary vascular networks into larger blood vessels during early development of the cardiovascular system (Dumont et al., Science 282, 946-949, 1998). Consistent with the lymphatic expression of Flt4 in adults overexpression of VEGF-C in the skin of transgenic mice resulted in lymphatic, but not vascular, endothelial proliferation and vessel enlargement (Jeltsch et al., Science 276, 1423-1425, 1997). Moreover, VEGF-C was reported to induce neovascularization in mouse cornea and chicken embryo chorioallantoic membrane models of angiogenesis (Cao et al., Proc. Natl. Acad. Sci. USA 95, 14389-14394, 1998).

The second class of endothelial cell specific receptor tyrosine kinases has also been found to be critically involved in the formation and integrity of vasculature. Mice deficient in Tie1 die of edema and hemorrhage resulting from poor structural integrity of endothelial cells of the microvasculature (Sato et al., Nature 376, 70-74, 1995; Rodewald & Sato, Oncogene 12, 397-404, 1996). The Tie2 knock-out phenotype is characterized by immature vessels lacking branching networks and lacking periendothelial support cells (Sato et al., Nature 376, 70-74, 1995; Dumont et al., Genes Dev. 8, 1897-1909, 1994). Targeted inactivation of the Tie2 ligand Ang1, as well as overexpression of Ang2, an inhibitory ligand, resulted in phenotypes similar to the Tie2 knock out (Maisonpierre et al., Science 277, 55-60, 1997; Suri et al., cell 87, 1171-1180). Conversely, increased vascularization was observed upon transgenic overexpression of Ang1 (Suri et al., Science 282, 468-471, 1998; Thurstonen et al., Science 286, 2511-2514, 1999).

The results from angiogenic growth factor expression studies in corpus luteum development (Maisonpierre et al., Science 277, 55-60, 1997; Goede et al. Lab. Invest. 78, 1385-1394, 1998), studies on blood vessel maturation in the retina (Alon et al., Nature Med. 1, 1024-1028, 1995; Benjamin et al, Development 125, 1591-1598, 1998), and gene targeting and transgenic experiments on Tie2, Ang1, and Ang2, suggest a fundamental role of the Angiopoietin/Tie receptor system in mediating interactions between endothelial cells and surrounding pericytes or smooth muscle cells. Ang1, which is expressed by the periendothelial cells and seems to be expressed constitutively in the adult, is thought to stabilize existing mature vessels. Ang2, the natural antagonist of Ang1 which is expressed by endothelial cells at sites of vessel sprouting, seems to mediate loosening of endothelial-periendothelial cell contacts to allow vascular remodeling and sprouting in cooperation with angiogenesis initiators such as VEGF, or vessel regression in the absence of VEGF (Hanahan, Science 277, 48-50, 1997).

In pathological settings associated with aberrant neovascularization elevated expression of angiogenic growth factors and of their receptors has been observed. Most solid tumors express high levels of VEGF and the VEGF receptors appear predominantly in endothelial cells of vessels surrounding or penetrating the malignant tissue (Plate et al., Cancer Res. 53, 5822-5827, 1993). Interference with the VEGF/VEGF receptor system by means of VEGF-neutralizing antibodies (Kim et al., Nature 362, 841-844, 1993), retroviral expression of dominant negative VEGF receptor variants (Millauer et al., Nature 367, 576-579, 1994), recombinant VEGF-neutralizing receptor variants (Goldman et al., Proc. Natl. Acad. Sci. USA 95, 8795-8800, 1998), or small molecule inhibitors of VEGF receptor tyrosine kinase (Fong et al., Cancer Res. 59, 99-106, 1999; Wedge et al., Cancer Res. 60, 970-975, 2000; Wood et al. Cancer Res. 60, 2178-2189, 2000), or targeting cytotoxic agents via the VEGF/VEGF receptor system (Arora et al., Cancer Res. 59, 183-188, 1999; EP 0696456A2) resulted in reduced tumor growth and tumor vascularization. However, although many tumors were inhibited by interference with the VEGF/VEGF receptor system, others were unaffected (Millauer et al., Cancer Res. 56, 1615-1620, 1996). Human tumors as well as experimental tumor xenografts contain a large number of immature blood vessels that have not yet recruited periendothelial cells. The fraction of immature vessels is in the range of 40% in slow growing prostate cancer and 90% in fast growing glioblastoma. A selective obliteration of immature tumor vessels was observed upon withdrawal of VEGF by means of downregulation of VEGF transgene expression in a C6 glioblastoma xenograft model. This result is in accordance with a function of VEGF as endothelial cell survival factor. Similarly, in human prostate cancer shutting off VEGF expression as a consequence of androgen-ablation therapy led to selective apoptotic death of endothelial cells in vessels lacking periendothelial cell coverage. In contrast, the fraction of vessels which resisted VEGF withdrawal showed periendothelial cell coverage (Benjamin et al., J. Clin. Invest. 103, 159-165, 1999).

The observation of elevated expression of Tie receptors in the endothelium of metastatic melanomas (Kaipainen et al., Cancer Res. 54, 6571-6577, 1994), in breast carcinomas (Salvén et al., Br. J. Cancer 74, 69-72, 1996), and in tumor xenografts grown in the presence of dominant-negative VEGF receptors (Millauer et al., Cancer Res. 56, 1615-1620, 1996), as well as elevated expression of Flt4 receptors in the endothelium of lymphatic vessels surrounding lymphomas and breast carcinomas (Jussila et al., Cancer Res. 58, 1599-1604, 1998), and of VEGF-C in various human tumor samples (Salvén et al., Am. J. Pathol. 153, 103-108, 1998), suggested these endothelium-specific growth factors and receptors as candidate alternative pathways driving tumor neovascularization. The high upregulation of Ang2 expression already in early tumors has been interpreted in terms of a host defense mechanism against initial cooption of existing blood vessels by the developing tumor. In the absence of VEGF, the coopted vessels undergo regression leading to necrosis within the center of the tumor. Contrarily, hypoxic upregulation of VEGF expression in cooperation with elevated Ang2 expression rescues and supports tumor vascularization and tumor growth at the tumor margin (Holash et al., Science 284, 1994-1998, 1999; Holash et al., Oncogene 18, 5356-5362, 1999).

Interference with Tie2 receptor function by means of Angiopoietin-neutralizing Tie2 variants consisting of the extracellular ligand-binding domain has been shown to result in inhibition of growth and vascularization of experimental tumors (Lin et al., J. Clin. Invest. 103, 159-165, 1999; Lin et al. Proc. Natl. Acad. Sci. USA 95, 8829-8834, 1998; Siemeister et al., Cancer Res. 59, 3185-3191, 1999). Comparing the effects of interference with the endothelium-specific receptor tyrosine kinase pathways by means of paracrine expression of the respective extracellular receptor domains on the same cellular background demonstrated inhibition of tumor growth upon blockade of the VEGF receptor system and of the Tie2 receptor system, respectively (Siemeister et al., Cancer Res. 59, 3185-3191, 1999).
It is known that the inhibition of the VEGF/VEGR receptor system by various methods resulted only in slowing down growth of most experimental tumors (Millauer et al., Nature 367, 576-579, 1994; Kim et al., Nature 362, 841-844, 1993; Millauer et al., Cancer Res. 56, 1615-1620, 1996; Goldman et al., Proc. Natl. Acad. Sci. USA 95, 8795-8800, 1998; Fong et al., Cancer Res. 59, 99-106, 1999; Wedge et al., Cancer Res. 60, 970-975, 2000; Wood et al. Cancer Res. 60, 2178-2189, 2000; Siemeister et al., Cancer Res. 59, 3185-3191, 1999). Even by escalation of therapeutic doses a plateau level of therapeutic efficacy was achieved (Kim et al., Nature 362, 841-844, 1993; Wood et al. Cancer Res. 60, 2178-2189, 2000). Similar results were observed upon interference with the Angiopoietin/Tie2 receptor system (Lin et al., J. Clin. Invest. 103, 159-165, 1999; Lin et al., Proc. Natl. Acad. Sci. USA 95, 8829-8834, 1998; Siemeister et al., Cancer Res. 59, 3185-3191, 1999).

However, there is a high demand for methods that enhance the therapeutic efficacy of anti-angiogenous compounds.

Searching for methods that enhance the therapeutic efficacy of anti-angiogenic compounds, superior anti-tumor effects were observed unexpectedly upon combination of inhibition of VEGF/VEGF receptor systems and interference with biological function of Angiopoietin/Tie receptor systems. The mode of action underlying the superior effects observed may be that interference biological function of Angiopoietin/Tie receptor systems destabilizes endothelial cell-periendothelial cell interaction of existing mature tumor vessels and thereby sensitizes the endothelium to compounds directed against VEGF/VEGF receptor systems.

Based on this unexpected finding the present invention provides the combination of functional interference with VEGF/VEGF receptor systems and with Angiopoietin/Tie receptor systems for inhibition of vascularization and of tumor growth.
The pharmaceutical composition consists of two components: compound I inhibits the biological activity of one or several of the VEGF/VEGF receptor systems or consists of cytotoxic agents which are targeted to the endothelium via recognition of VEGF/VEGF receptor systems. Compound II interferes with the biological function of one or several of Angiopoietin/Tie receptor systems or consists of cytotoxic agents which are targeted to the endothelium via recognition of Angiopoietin/Tie receptor systems. Alternatively, compound I inhibits the biological activity of one or several of the VEGF/VEGF receptor systems or of the Angiopoietin/Tie receptor systems and coumpound II consists of cytotoxic agents which are targeted to the endothelium via recognition of one or several of the VEGF/VEGF receptor systems or of the Angiopoietin/Tie receptor systems. Targeting or modulation of the biological activities of VEGF/VEGF receptor systems and of Angiopietin/Tie receptor systems can be performed by
(a) compounds which inhibit receptor tyrosine kinase activity,
(b) compounds which inhibit ligand binding to receptors,
(c) compounds which inhibit activation of intracellular signal pathways of the receptors,
(d) compounds which inhibit or activate expression of a ligand or of a receptor of the VEGF or Tie receptor system,
(e) delivery systems, such as antibodies, ligands, high-affinity binding oligonucleotides or oligopeptides, or liposomes, which target cytotoxic agents or coagulation-inducing agents to the endothelium via recognition of VEGF/VEGF receptor or Angiopoietin/Tie receptor systems,
(f) delivery systems, such as antibodies, ligands, high-affinity binding oligonucleotides or oligopeptides, or liposomes, which are targeted to the endothelium and induce necrosis or apoptosis.

A compound comprised by compositions of the present invention can be a small molecular weight substance, an oligonucleotide, an oligopeptide, a recombinant protein, an antibody, or conjugates or fusionproteins thereof. An example of an inhibitor is a small molecular weight molecule which inactivates a receptor tyrosine kinase by binding to and occupying the catalytic site such that the biological activity of the receptor is decreased. Kinase inhibitors are known in the art (Sugen: SU5416, SU6668; Fong et al. (1999), Cancer Res. 59, 99-106; Vajkoczy et al., Proc. Am. Associ. Cancer Res. San Francisco (2000), Abstract ID 3612; Zeneca: ZD4190, ZD6474; Wedge et al. (2000), Cancer Res. 60, 970-975; Parke-Davis PD0173073, PD0173074; Johnson et al., Proc. Am. Associ. Cancer Res., San Franzisco (2000), Abstract ID 3614; Dimitroff et al. (1999), Invest. New Drugs 17, 121-135). An example of an antagonist is a recombinant protein or an antibody which binds to a ligand such that activation of the receptor by the ligand is prevented. Another example of an antagonist is an antibody which binds to the receptor such that activation of the receptor is prevented. An example of an expression modulator is an antisense RNA or ribozyme which controls expression of a ligand or a receptor. An example of a targeted cytotoxic agent is a fusion protein of a ligand with a bacterial or plant toxin such as Pseudomonas exotoxin A, Diphtheria toxin, or Ricin A. An example of a targeted coagulation-inducing agent is a conjugate of a single chain antibody and tissue factor. Ligand-binding inhibitors such as neutralizing antibodies which are known in the art are described by Genentech (rhuMAbVEGF) and by Presta et al. (1997), Cancer Res. 57, 4593-4599. Ligand-binding receptor domaines are described by Kendall & Thomas (1993), Proc. Natl. Acad. Sci., U.S.A.90, 10705-10709; by Goldman et al. (1998) Proc. Natl. Acad. Sci., U.S.A.95, 8795-8800 and by Lin et al. (1997), J. Clin. Invest. 100, 2072-2078. Further, dominant negative receptors have been described by Millauer et al. (1994), Nature 367, 567-579.
Receptor blocking antibodies have been described by Imclone (c-p1C11, US 5,874,542). Further known are antagonistic ligand mutants (Siemeister et al. (1998), Proc. Natl. Acad. Sci., U.S.A.95, 4625-4629). High affinity ligand- or receptor binding oligo nucleotides habe been described by NeXstar (NX-244) and Drolet et al. (1996), Nat. Biotech 14, 1021-1025. Further, small molecules and peptides have been described.

Expression regulators have been described as anti-sense oligo nucleotides and as ribozymes (RPI, Angiozyme™, see RPI Homepage).

Examples for delivery-/Targeting-Systems have been described as ligand/ antibody-toxin-fusion-proteins or conjugates (Arora et al. (1999), Cancer Res. 59, 183-188 and Olson et al. (1997), Int. J. Cancer 73, 865-870), as endothel cell targeting of liposomes (Spragg et al. (1997), Prog. Natl. Acad. Sci, U.S.A94, 8795-8800, and as endothel cell targeting plus coagulation-induction (Ran et al., (1998), Cancer Res. 58, 4646-4653).

Small molecules which inhibit the receptor tyrosine kinase activity are for example molecules of general formula I 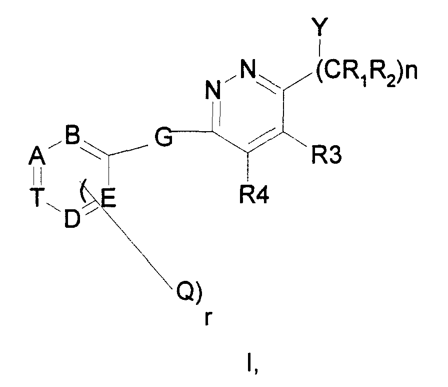 in which
- r: has the meaning of 0 to 2,
- n: has the meaning of 0 to 2;
- R₃ und R₄: a) each independently from each other have the meaning of lower alkyl,
b) together form a bridge of general partial formula II, 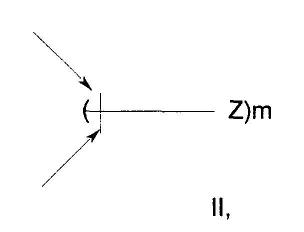
wherein the binding is via the two terminal C- atoms, and
- m: has the meaning of 0 to 4; or
c) together form a bridge of partial formula III 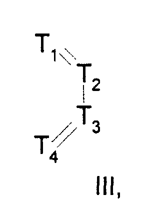 wherein one or two of the ring members T₁,T₂,T₃,T₄ has the meaning of nitrogen, and each others have the meaning of CH, and the bining is via the atoms T₁ and T₄;
- G: has the meaning of C₁ -C₆ - alkyl, C₂ - C₆ - alkylene or C₂ - C₆ - alkenylene; or C₂ - C₆- alkylene or C₃ -C₆- alkenylene, which are substituted with acyloxy or hydroxy; -CH₂-O-, -CH₂-S-, -CH₂-NH-, -CH₂-O-CH₂-, -CH₂-S-CH₂-, -CH₂-NH-CH₂, oxa (-O-), thia (-S-) or imino (-NH-),
- A, B, D, E and T: independently from each other have the meaning of N or CH, with the provisio that not more than three of these Substituents have the meaning of N,
- Q: has the meaning of lower alkyl, lower alkyloxy or halogene,
- R₁ and R₂: independently from each other have the meaning of H or lower alkyl,
- X: has the meaning of imino, oxa or thia;
- Y: has the meaning of hydrogene, unsubstituted or substituted aryl, heteroaryl, or unsubstituted or substituted cycloalkyl; and
- Z: has the meaning of amino, mono- or disubstituted amino, halogen, alkyl, substituted alkyl, hydroxy, etherificated or esterificated hydroxy, nitro, cyano, carboxy, esterificated carboxy, alkanoyl, carbamoyl, N-mono- or N, N- disubstituted carbamoyl, amidino, guanidino, mercapto, sulfo, phenylthio, phenyl-lower-alkyl-thio, alkyl-phenyl-thio, phenylsulfinyl, phenyl-lower-alkyl-sulfinyl, alkylphenylsulfinyl, phenylsulfonyl, phenyl-lower-alkan-sulfonyl, or alkylphenylsulfonyl, whereas, if more than one rest Z is present (m≥2), the substituents Z are equal or different from each other, and wherein the bonds marked with an arrow are single or double bonds; or an N-oxide of said compound, wherein one ore more N-atoms carry an oxygene atom, or a salt thereof.

A preferred salt is the salt of an organic acid, especially a succinate.

These compounds can preferentially be used as compound I or II in the inventive pharmaceutical composition.

Compounds which stop a tyrosin phosphorylation, or the persistent angiogenese, respectively, which results in a prevention of tumor growth and tumor spread, are for example
anthranyl acid derivatives of general formula IV 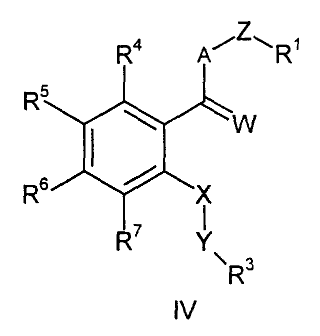 in which
- A: has the meaning of group =NR²,
- W: has the meaning of oxygen, sulfur, two hydrogen atoms or the group =NR⁸,
- Z: has the meaning of the group =NR¹⁰ or =N-, -N(R¹⁰)-(CH₂)_{q}-, branched or unbranched C₁₋₆-Alkyl or is the group 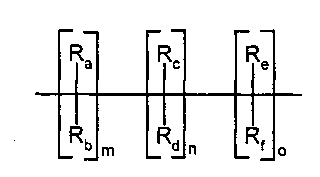 or A, Z and R¹ together form the group 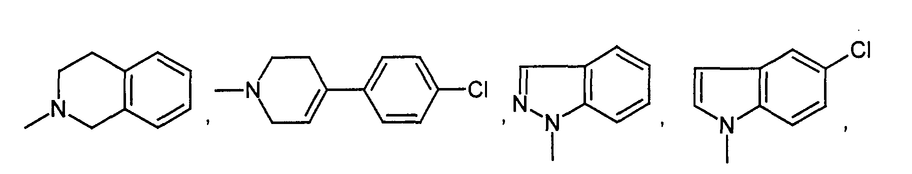 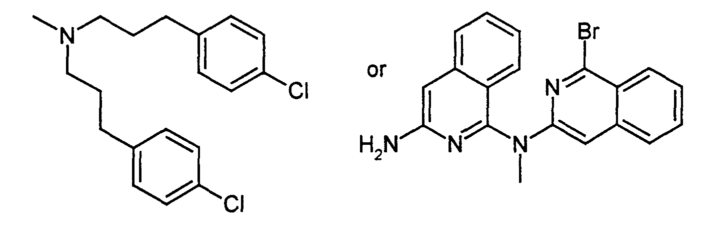
- m, n and o: has the meaning of 0 - 3,
- q: has the meaning of 1 - 6,
- Rₐ, R_{b}, R_{c}, R_{d}, Rₑ, R_{f}: independently from each other have the meaning of hydrogen, C₁₋₄ alkyl or the group =NR¹⁰, and/ or Rₐ and/ or R_{b} together with R_{c} and or R_{d} or R_{c} together with Rₑ and/ or R_{f} form a bound, or up to two of the groups Rₐ-R_{f} form a bridge with each up to 3 C-atoms with R¹ or R²,
- X: has the meaning of group =NR⁹ or =N-,
- Y: has the meaning of group -(CH₂)ₚ,
- P: has the meaning of integer 1-4,
- R¹: has the meaning of unsubstituted or optionally substituted with one or more of halogene, C₁₋₆-alkyl, or C₁₋₆-alkyl or C₁₋₆-alkoxy, which is optionally substituted by one or more of halogen, or is unsubstituted or substituted aryl or heteroaryl,
- R²: has the meaning of hydrogen or C₁₋₆-alkyl, or form a bridge with up to 3 ring atoms with Rₐ-R_{f} together with Z or R₁,
- R³: has the meaning of monocyclic or bicyclic aryl or heteroaryl which is unsubstituted or optionally substituted with one or more of für halogen, C₁₋₆-alkyl, C₁₋₆-alkoxy or hydroxy,
- R⁴ ,R⁵, R⁶ and R⁷: independently from each other have the meaning of hydrogen, halogen or C₁₋₆-alkoxy, C₁₋₆-alkyl or C₁₋₆-carboxyalkyl, which are unsubstituted or optionally substituted with one or more of halogen, or R⁵ and R⁶ together form the group 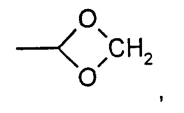
- R⁸, R⁹ and R¹⁰: independently from each other have the meaning of hydrogen or C₁₋₆-alkyl, as well as their isomers and salts.

These compounds can also preferentially be used as compound I or II in the inventive pharmaceutical composition.

More preferentially compounds of genearal formula V 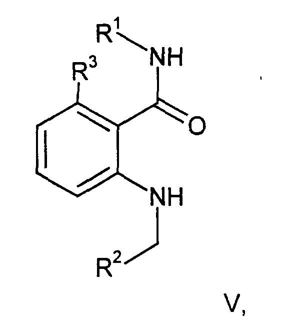 in which
R¹ has the meaning of group 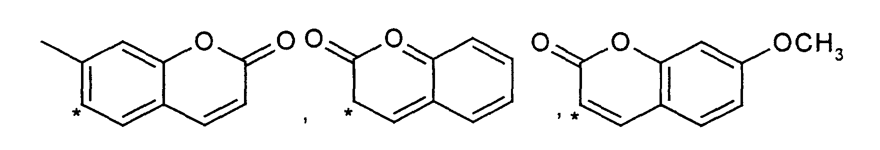 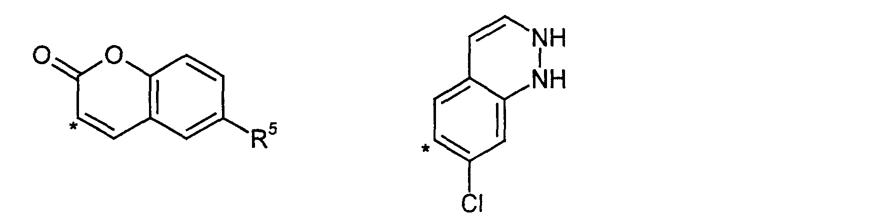 in which R⁵ is chloro, bromo or the group -OCH₃, 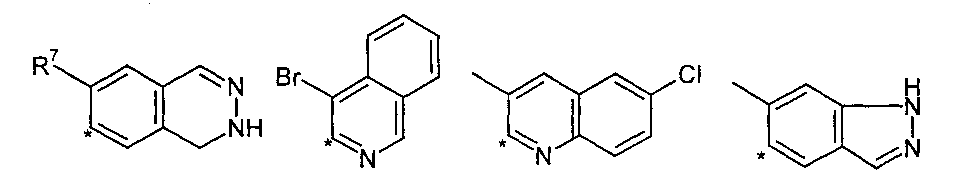 in which R⁷ is -CH₃ or chloro,

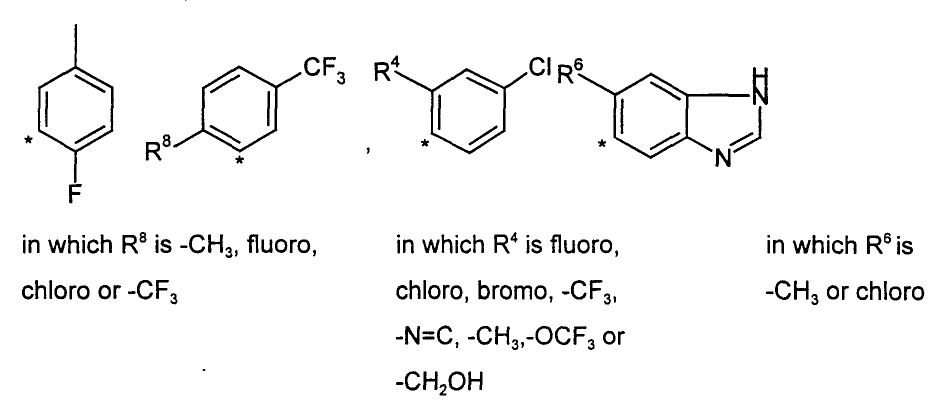

R² has the meaning of pyridyl or the group 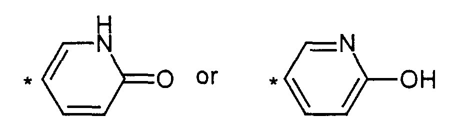 and
R³ has the meaning of hydrogen or fluoro, as well as their isomers and salts can be used as compound I or II in the inventive pharmaceutical composition.

These compounds have the same properties as already mentioned above under compound IV and can be used for the treatment of angiogeneous diseases. Compositions comprise compounds of general formulars I, IV and V, alone or in combination.
The above mentioned compounds are also claimed matter within the inventive combinations.

A further example for ligand binding inhibitors are peptides and DNA sequences coding for such peptides, which are used for the treatment of angiogeneous diseases. Such peptides and DNA sequences are disclosed in Seq. ID No. 1 to 59 of the sequence protocoll. It has been shown that Seq. ID Nos. 34 and 34a are of main interest.

Claimed matter of the instant invention are therefor pharmaceutical compositions
a) comprising one or several agents as compound I which modulate the biological function of one or several of the VEGF/VEGF receptor systems, and comprising one or several agents as compound II which modulate the biological function of one or several of the Angiopoietin/Tie receptor systems,
b) comprising one or several agents as compound I which are targeted to the endothelium via of one or several of the VEGF/VEGF receptor systems, and comprising one or several agents as compound II which modulate the biological function of one or several of the Angiopoietin/Tie receptor systems,
c) comprising one or several agents as compound I which modulates the biological function of one or several of the VEGF/VEGF receptor systems or of one or several of the Angiopoietin/ Tie receptor systems and comprising one or several agents as compound II which are targeted to the endothelium,
d) comprising one or several agents as compound I which modulate the biological function of one or several of the VEGF/VEGF receptor systems, and comprising one or several agents as compound II which are targeted to the endothelium via one or several of the Angiopoietin/Tie receptor systems,
e) comprising one or several agents as compound I which are targeted to the endothelium via one or several of the VEGF/VEGF receptor systems, and comprising one or several agents as compound II which are targeted to the endothelium via one or several of the Angiopoietin/Tie receptor systems,
f) comprising one or several agents as compound I which modulate the biological function of one or several of the VEGF/VEGF receptor systems, and comprising one or several agents as compound II which are targeted to the endothelium via one or several of the VEGF/VEGF receptor systems,
g) comprising one or several agents as compound I which modulate the biological function of one or several of the Angiopoietin/Tie receptor systems, and comprising one or several agents as compound II which are targeted to the endothelium via one or several of the Angiopoietin/Tie receptor systems and
h) comprising one or several agents which interfere with both the function of one or several of the VEGF/VEGF receptor systems and the function of one or several of the Angiopoietin/Tie receptor systems.

For a sequential therapeutical application the inventive pharmaceutical compositions can be applied simultaneously or separately .

The inventive compositions comprise as compound I or as compound II at least one of
a) compounds which inhibit receptor tyrosine kinase activity,
b) compounds which inhibit ligand binding to receptors,
c) compounds which inhibit activation of intracellular signal pathways of the receptors,
d) compounds which inhibit or activate expression of a ligand or of a receptor of the VEGF or Tie receptor system,
e) delivery systems, such as antibodies, ligands, high-affinity binding oligonucleotides or oligopeptides, or liposomes, which target cytotoxic agents or coagulation-inducing agents to the endothelium via recognition of VEGF/VEGF receptor or Angiopoietin/Tie receptor systems,
f) delivery systems, such as antibodies, ligands, high-affinity binding oligonucleotides or oligopeptides, or liposomes, which are targeted to the endothelium and induce necrosis or apoptosis.
These compositions are also claimed matter of the present invention.

Also claimed matter of the present invention are pharmaceutical compositions which comprise as compound I and/ or II at least one of Seq. ID Nos. 1-59. Of most value are pharmaceutical compositions, which comprise as compound I and/ or II Seq. ID Nos. 34a und pharmaceutical compositions according to claims which comprise as compound I and/ or II at least one of sTie2, mAB 4301-42-35 and/ or scFv-tTF conjugate.

Further preferred matter of the present invention are pharmaceutical compositions, which comprise as compound I and/ or II at least one small molecule of general formula I, general formula IV and/ or general formula V.

The most preferred compound which can be used as compound I or II in the inventive composition is (4-Chlorophenyl)[4-(4-pyridylmethyl)-phthalazin-1-yl]ammonium hydrogen succinate.
Therefore, claimed matter of the present invention are also pharmaceutical compositions, which comprise as compound I (4-Chlorophenyl)[4-(4-pyridylmethyl)-phthalazin-1-yl]ammonium hydrogen succinate, sTie2, mAB 4301-42-35 and/ or scFv-tTF conjugate.and as compound II (4-Chlorophenyl)[4-(4-pyridylmethyl)-phthalazin-1-yl]ammonium hydrogen succinatesTie2, mAB 4301-42-35 and/ or scFv-tTF conjugate, with the provisio that compound I is not identically to compound II, and most preferred pharmaceutical compositions, which comprise as compound I (4-Chlorophenyl)[4-(4-pyridylmethyl)-phthalazin-1-yl]ammonium hydrogen succinate and as compound II sTie2, mAB 4301-42-35 and/ or scFv-tTF conjugate; pharmaceutical compositions, which comprise as compound I mAB 4301-42-35 and as compound II sTie2, and/ or scFv-tTF conjugate; pharmaceutical compositions, which comprise as compound I scFv-tTF conjugate and as compound II sTie2 and/ or mAB 4301-42-35.

The small molecule compounds, proteins and DNA's expressing proteins, as mentioned above can be used as medicament alone, or in form of formulations for the treatment of tumors, cancers, psoriasis, arthritis, such as rheumatoide arthritis, hemangioma, angiofribroma, eye diseases, such as diabetic retinopathy, neovascular glaukoma, kidney diseases, such as glomerulonephritis, diabetic nephropathy, maligneous nephrosclerose, thrombic microangiopatic syndrome, transplantation rejections and glomerulopathy, fibrotic diseases, such as cirrhotic liver, mesangial cell proliferative diseases, artheriosclerosis and damage of nerve tissues.
The treatment of the damaged nerve tissues with the inventive combination hinders the rapid formation of scars at the damaged position. Thus, there is no scar formation before the axons communicate with each other. Therefore a reconstruction of the nerve bindings is much more easier.

Further, the inventive combinations can be used for suppression of the ascites formation in patients. It is also possible to suppress VEGF oedemas.
For the use of the inventive combinations as medicament the compounds will be formulated as pharmaceutical composition. Said formulation comprises beside the active compound or compounds acceptable pharmaceutically, organically or inorganically inert carriers, such as water, gelatine, gum arabic, lactose, starch, magnesium stearate, talcum, plant oils, polyalkylene glycols, etc. Said pharmaceutical preparations can be applied in solid form, such as tablets, pills, suppositories, capsules, or can be applied in fluid form, such as solutions, suspensions or emulsions.
If necessary, the compositions additionally contain additives, such as preservatives, stabilizer, detergents or emulgators, salts for alteration of the osmotic pressure and/ or buffer.
These uses are also claimed matter of the instant invention, as well as the formulations of the active compounds

For parenteral application especially injectable solutions or suspensions are suitable, especially hydrous solutions of the active compound in polyhydroxyethoxylated castor-oil are suitable.
As carrier also additives can be used, such as salts of the gallic acid or animal or plant phospholipids, as well as mixtures thereof, and liposomes or ingredients thereof.
For oral application especially suitable are tablets, pills or capsules with talcum and/ or hydrocarbon carriers or binders, such as lactose, maize or potato starch.

The oral application can also be in form of a liquid, such as juice, which optionally contains a sweetener.
The dosis of the active compound differs depending on the application of the compound, age and weight of the patient, as well as the form and the progress of the disease.
The daily dosage of the active compound is 0,5-1000 mg, especially 50-200 mg. The dosis can be applied as single dose or as two or more daily dosis.

These formulations and application forms are also part of the instant invention.

Combined functional interference with VEGF/VEGF receptor systems and with Angiopoietin/Tie receptor systems can be performed simultaneously, or in sequential order such that the biological response to interference with one ligand/receptor system overlaps with the biological response to interference with a second ligand/receptor system. Alternatively, combined functional interference with VEGF/VEGF receptor systems or with Angiopoietin/Tie receptor systems and targeting of cytotoxic agents via VEGF/VEGF receptor systems or via Angiopoietin/Tie receptor systems can be performed simultaneously, or in sequential order such that the biological response to functional interference with a ligand/receptor system overlaps in time with targeting of cytotoxic agents.

The invention is also directed to a substance which functional interferes with both VEGF/VEGF receptor systems and Angiopoietin/Tie receptor systems, or which are targeted via both VEGF/VEGF receptor systems and Angiopoietin/Tie receptor systems.

VEGF/VEGF receptor systems include the ligands VEGF-A, VEGF-B, VEGF-C, VEGF-D, PIGF, and the receptor tyrosine kinases VEGF-R1 (Flt1), VEGF-R2 (KDR/Flk1), VEGF-R3 (Flt4), and their co-receptors (i.e. neuropilin-1). Angiopoietin/Tie receptor systems include Ang1, Ang2, Ang3/Ang4, and angiopoietin related polypeptides which bind to Tie1 or to Tie2, and the receptor tyrosine kinases Tie1 and Tie2.

Phamaceutical compositions of the present invention can be used for medicinal purposes. Such diseases are, for example, cancer, cancer metastasis, angiogenesis including retinopathy and psoriasis. Pharmaceutical compositions of the present invention can be applied orally, parenterally, or via gene therapeutic methods.

Therefor the present invention also concerns the use of pharmaceutical compositions for the production of a medicament for the treatment of tumors, cancers, psoriasis, arthritis, such as rheumatoide arthritis, hemangioma, angiofribroma, eye diseases, such as diabetic retinopathy, neovascular glaukoma, kidney diseases, such as glomerulonephritis, diabetic nephropathie, maligneous nephrosclerosis, thrombic microangiopatic syndrome, transplantation rejections and glomerulopathy, fibrotic diseases, such as cirrhotic liver, mesangial cell proliferative diseases, artheriosclerosis, damage of nerve tissues, suppression of the ascites formation in patients and suppression of VEGF oedemas.

The following example demonstrates the feasability of the disclosed invention, without restricting the inventon to the disclosed example.

### Example 1

Superior effect on inhibition of tumor growth via combination of inhibition of the VEGF A/VEGF receptor system together with functional interference with the Angiopoietin/Tie2 receptor system over separate modes of intervention was demonstrated in an A375v human melanoma xenograft model.

Human melanoma cell line A375v was stably transfected to overexpress the extracellular ligand-neutralizing domain of human Tie2 receptor tyrosine kinase (sTie2; compound II) (Siemeister et al., Cancer Res. 59, 3185-3191, 1999). For control, A375v cells were stably transfected with the empty expression vector (A375v/pCEP). Swiss *nu/nu* mice were s.c. injected with 1x10⁶ transfected A375v/sTie2 or A375v/pCEP tumor cells, respectively. Animals receiving compound I were treated for up to 38 days with daily oral doses of 50 mg/kg of the VEGF receptor tyrosine kinase inhibitor (4-Chlorophenyl)[4-(4-pyridylmethyl)-phthalazin-1-yl]ammonium hydrogen succinate (Wood et al., Cancer Res. 60, 2178-2189, 2000). Various modes of treatment are described in Table 1. Tumor growth was determined by caliper measurement of the largest diameter and its perpendicular.

**Table 1**

| | mode of treatment | |
|---|---|---|
| treatment group | (4-Chlorophenyl)[4-(4-pyridylmethyl)-phthalazin-1-yl]ammonium hydrogen succinate (compound I) | sTie2 (compound II) |
| Group 1: A375v/pCEP | - | - |
| Group 2: A375v/pCEP | + | - |
| Group 3: A375v/sTie2 | - | + |
| Group 4: A375v/sTie2 | + | + |

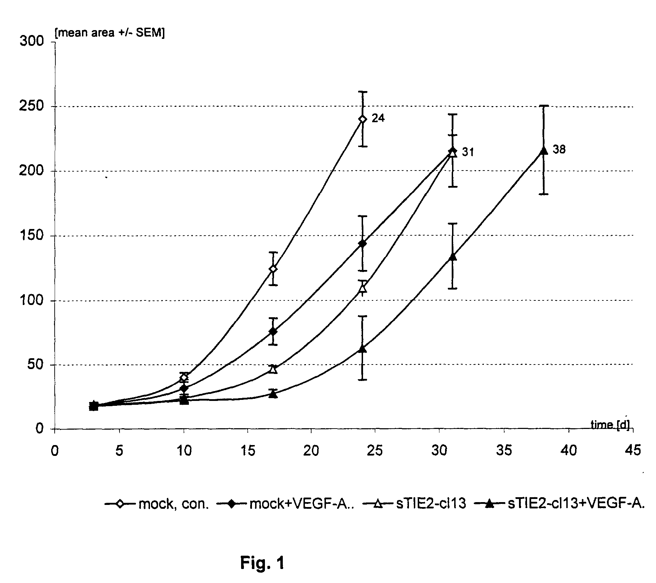

Tumors derived from A375v/pCEP control cells reached a size of approx. 250 mm² (mean area) within 24 days (Figure 1) without treatment (group 1). Separate treatment with the VEGF receptor inhibitor (4-Chlorophenyl)[4-(4-pyridylmethyl)-phthalazin-1-yl]ammonium hydrogen succinate (compound I, treatment group 2) or separate interference with Angiopoietin/Tie2 receptor system by means of expression of sTie2 (compound II, treatment group 3) delayed growth of tumors to a size of approx. 250 mm² to 31 days, respectively. Combination of interference with the Angiopoietin/Tie2 system by means of expression of sTie2 and of interference with the VEGF/VEGF receptor system by means of the kinase inhibitor (4-Chlorophenyl)[4-(4-pyridylmethyl)-phthalazin-1-yl]ammonium hydrogen succinate (compound I + compound II, treatment group 4) delayed growth of the tumors to a size of approx. 250 mm² to 38 days. This result clearly demonstrates the superior effect of a combination of interference with the VEGF-A/VEGF receptor system and the Angiopoietin/Tie2 receptor system over separate modes of intervention.

### Description of the figures

Fig. 1 shows the superior effect of combination of interference with VEGF/VEGF receptor system by means of an specific tyrosine kinase inhibitor and with the Angiopoietin/Tie2 receptor system by means of a soluble receptor domain on inhibition of tumor growth (treatment modes of groups 1-4 are given in Table 1).
The abbreviations have the following meaning:
- mock, con. =: treatment group 1
- mock+VEGF-A =: treatment group 2
- sTIE2-c113 =: treatment group 3
- sTIE2-c113+VEGF-A =: treatment group 4

Sequenzprotokoll

<110> Schering Aktiengesellschaft

<120> Combinations and compositions which interfere with VEGF/ VEGF and
angiopoietin/ Tie receptor function and their use

<130> 51849AEPM1XX00-P

<140>
<141>

<160> 59

<210> 1
<211> 1835
<212> DNA
<213> Human

<400> 1

ttttacagtt ttccttttct tcagagttta ttttgaattt tcatttttgg ataaccaagc 60
agctctttaa gaagaatgca cagaagagtc attctggcac ttttggatag tacataagat 120
tttctttttt ttttttaaat tttttttaat agtcacattc agctcgcttg ctcaaaccag 180
actcccacat tgggtgagca agatgagccc ataggattcc agagttaata cgtaaccgta 240
tatacaaaca gccaaaaaac cataatggtg ccacagggat ggagcaggga agggcatctc 300
taacgtgtcc tctagtctat cttcgctaaa cagaacccac gttacacatg ataactagag 360
agcacactgt gttgaaacga ggatgctgac cccaaatggc acttggcagc atgcagttta 420
aagcaaaaga gacatccttt aataactgta taaaatccag gcagttccat taaaggggtt 480
aagaaaacca acaacaacaa aaagcgaggg actgtctgtt gtcactgtca aaaaggcact 540
tggagttaat gggaccagga ttggaggact cttagctgat acagatttca gtacgatttc 600
attaaaaggc ttggatgtta agagaggaca ctcagcggtt cctgaaggga gacgctgaga 660
tggaccgctg agaagcggaa cagatgaaca caaaggaatc aaatctttac aaccaaattg 720
catttaagcg acaacaaaaa aaggcaaacc ccaaaacgca acctaaccaa agcaaaatct 780
aagcaaaatc agacaacgaa gcagcgatgc atagctttcc tttgagagaa cgcatacctt 840
gagacgctac gtgccaacct aagttctcaa cgacagcttc acagtaggat tattgtgata 900
aaaatgactc aagcgatgca aaaagtttca tctgttccca gaatccgagg gagaactgag 960
gtgatcgtta gagcatagcg acatcacgtg cggtttctta atgtccctgg tggcggatac 1020
gccgagtcct cggaaggaca tctggacacc actttcagcc acctccttgc aggggcgaca 1080
tccgccaaag tcatccttta ttccgagtaa taactttaat tcctttctaa catttacacg 1140
gcaaacagga atgcagtaaa cgtccacgtc cgtcccacgg ctgggctgcc gttccgtttc 1200
ctccacgaac gggtacgcgc ttccatgaga aaggatattt ggcaatttta tattccacag 1260
tcaggtgggt ctgcgatagc tcatttaatg ttaaacgcca tcaggggcct ctcctcccgt 1320
ttctgccagg ggcttttctt gtcttctcct tggcgagctc gtgggcagat cttctctggt 1380
gggggctggc tgctggctcc gagggggcat ccgcagtccg tctggtcgtc tcctcctgca 1440
ggctgggcag ctggccacca cttctccgac tcgacccctc caacaagcat cgcagggcac 1500
tgtcctcggg ggtacagacc gtggtcccac attcgctacc actctgttcc acgtcatcca 1560
ggtacacgag ctgcgtgtag gccgtgctgt ctggggctcg aggctctttc tgctggtgct 1620
cttggacggg cgggtagttc tgctgcagag acaaagcatc tccccttccc ttccgggctg 1680
attttggttc attcatatct acgccagagt ccaaactggc atcattactt ccgttccttc 1740
cagctctttg gagaatcaat gtatgaatgt ctaacctgac cgttggacct gccatccaag 1800
gagacgaacc acgcccgggg gtgcggaagc ggcct

<210> 2
<211> 581
<212> DNA
<213> Human

<400> 2

gttctagatt gttttattca gtaattagct cttaagaccc ctggggcctg tgctacccag 60
acactaacaa cagtctctat ccagttgctg gttctgggtg acgtgatctc cccatcatga 120

tcaacttact tcctgtggcc cattagggaa gtggtgacct cgggagctat ttgcctgttg 180
agtgcacaca cctggaaaca tactgctctc attttttcat ccacatcagt gagaaatgag 240
tggcccgtta gcaagatata actatgcaat catgcaacaa agctgcctaa taacatttca 300
tttattacag gactaaaagt tcattattgt ttgtaaagga tgaattcata acctctgcag 360
agttatagtt catacacagt tgatttccat ttataaaggc agaaagtcct tgttttctct 420
aaatgtcaag ctttgactga aaactcccgt ttttccagtc actggagtgt gtgcgtatga 480
aagaaaatct ttagcaatta gatgggagag aagggaaata gtacttgaaa tgtaggccct 540
cacctcccca tgacatcctc catgagcctc ctgatgtagt g

<210> 3
<211> 516
<212> DNA
<213> Human

<400> 3

tagagatgtt ggttgatgac ccccgggatc tggagcagat gaatgaagag tctctggaag 60
tcagcccaga catgtgcatc tacatcacag aggacatgct catgtcgcgg aacctgaatg 120
gacactctgg gttgattgtg aaagaaattg ggtcttccac ctcgagctct tcagaaacag 180
ttgttaagct tcgtggccag agtactgatt ctcttccaca gactatatgt cggaaaccaa 240
agacctccac tgatcgacac agcttgagcc tcgatgacat cagactttac cagaaagact 300
tcctgcgcat tgcaggtctg tgtcaggaca ctgctcagag ttacaccttt ggatgtggcc 360
atgaactgga tgaggaaggc ctctattgca acagttgctt ggcccagcag tgcatcaaca 420
tccaagatgc ttttccagtc aaaagaacca gcaaatactt ttctctggat ctcactcatg 480
atgaagttcc agagtttgtt gtgtaaagtc cgtctg

<210> 4
<211> 1099
<212> DNA
<213> Human

<400> 4

cccacaacac aggggccctg aaacacgcca gcctctcctc tgtggtcagc ttggcccagt 60
cctgctcact ggatcacagc ccattgtagg tggggcatgg tggggatcag ggcccctggc 120
ccacggggag gtagaagaag acctggtccg tgtaagggtc tgagaaggtg ccctgggtcg 180
ggggtgcgtc ttggccttgc cgtgccctca tcccccggct gaggcagcga cacagcaggt 240
gcaccaactc cagcaggtta agcaccaggg agatgagtcc aaccaccaac atgaagatga 300
tgaagatggt cttctccgtg gggcgagaga caaagcagtc cacgaggtag gggcagggtg 360
ctcgctggca cacaaacacg ggctccatgg tccagccgta caggcgccac tggccataga 420
ggaagcctgc ctctagcaca ctcttgcaga gcacactggc gacataggtg cccatcagtg 480
ctccgcggat gcgcaggcga ccatcttctg ccaccgagat cttggccatc tgacgctcta 540
cggccgccag cgcccgctcc acctgtgggt ccttggccgg cagtgcccgc agctccccct 600
ccttctgccg cagccgctct tctcgccgag acaggtaaat gacatggccc aggtagacca 660
gggtgggtgt gctgacgaag aggaactgca gcacccagta gcggatgtgg gagatgggga 720
aggcctggtc atagcagacg ttggtgcagc ctggctgggc cgtgttacac tcgaaatctg 780
actgctcgtc accccacact gactcgccgg ccaggcccag gatgaggatg cggaagatga 840
agagcaccgt cagccagatc ttacccacca cggtcgagtg ctcctggacc tggtccagca 900
acttctccac gaagccccag tcacccatgg ctcccgggcc tccgtcggca aggagacaga 960
gcacgtcagt gtgtcagcat ggcatccttc tcgttcgccc agcaacaagc ctgcagggag 1020
gtctgccacg cccgttctac cgcctgcctg ccgggcggcc caggtggagg tggggacgat 1080
ggccggagtg acgcccgcg

<210> 5
<211> 1015
<212> DNA
<213> Human

<400> 5

gaggataggg agcctggggt caggagtgtg ggagacacag cgagactctg tctccaaaaa 60
aaaaagtgct ttttgaaaat gttgaggttg aaatgatggg aaccaacatt ctttggattt 120
agtggggagc ataatagcaa acaccccctt ggttcgcaca tgtacaggaa tgggacccag 180
ttggggcaca gccatggact tccccgccct ggaatgtgtg gtgcaaagtg gggccagggc 240
ccagacccaa gaggagaggg tggtccgcag acaccccggg atgtcagcat cccccgacct 300
gccttctggc ggcacctccc gggtgctgtg ttgagtcagc aggcatgggg tgagagcctg 360

gtatatgctg ggaacagggt gcaggggcca agcgttcctc cttcagcctt gacttgggcc 420
atgcaccccc tctcccccaa acacaaacaa gcacttctcc agtatggtgc caggacaggt 480
gtcccttcag tcctctggtt atgacctcaa gtcctacttg ggccctgcag cccagcctgt 540
gttgtaacct ctgcgtcctc aagaccacac ctggaagatt cttcttccct ttgaaggaga 600
atcatcattg ttgctttatc acttctaaga cattttgtac ggcacggaca agttaaacag 660
aatgtgcttc cctccctggg gtctcacacg ctcccacgag aatgccacag gggccgtgca 720
ctgggcaggc ttctctgtag aaccccaggg gcttcggccc agaccacagc gtcttgccct 780
gagcctagag cagggagtcc cgaacttctg cattcacaga ccacctccac aattgttata 840
accaaaggcc tcctgttctg ttatttcact taaatcaaca tgctattttg ttttcactca 900
cttctgactt tagcctcgtg ctgagccgtg tatccatgca gtcatgttca cgtgctagtt 960
acgtttttct tcttacacat gaaaataaat gcataagtgt tagaagaaaa aaaaa

<210> 6
<211> 2313
<212> DNA
<213> Human

<400> 6

ccagagcagg cctggtggtg agcagggacg gtgcaccgga cggcgggatc gagcaaatgg 60
gtctggccat ggagcacgga gggtcctacg ctcgggcggg gggcagctct cggggctgct 120
ggtattacct gcgctacttc ttcctcttcg tctccctcat ccaattcctc atcatcctgg 180
ggctcgtgct cttcatggtc tatggcaacg tgcacgtgag cacagagtcc aacctgcagg 240
ccaccgagcg ccgagccgag ggcctataca gtcagctcct agggctcacg gcctcccagt 300
ccaacttgac caaggagctc aacttcacca cccgcgccaa ggatgccatc atgcagatgt 360
ggctgaatgc tcgccgcgac ctggaccgca tcaatgccag cttccgccag tgccagggtg 420
accgggtcat ctacacgaac aatcagaggt acatggctgc catcatcttg agtgagaagc 480
aatgcagaga tcaattcaag gacatgaaca agagctgcga tgccttgctc ttcatgctga 540
atcagaaggt gaagacgctg gaggtggaga tagccaagga gaagaccatt tgcactaagg 600
ataaggaaag cgtgctgctg aacaaacgcg tggcggagga acagctggtt gaatgcgtga 660
aaacccggga gctgcagcac caagagcgcc actggccaag gagcaactgc aaaaggtgca 720
agccctctgc ctgcccctgg acaaggacaa gtttgagatg gaccttcgta acctgtggag 780
ggactccatt atcccacgca gcctggacaa cctgggttac aacctctacc atcccctggg 840
ctcggaattg gcctccatcc gcagagcctg cgaccacatg cccagcctca tgagctccaa 900
ggtggaggag ctggcccgga gcctccgggc ggatatcgaa cgcgtggccc gcgagaactc 960
agacctccaa cgccagaagc tggaagccca gcagggcctg cgggccagtc aggaggcgaa 1020
acagaaggtg gagaaggagg ctcaggcccg ggaggccaag ctccaagctg aatgctcccg 1080
gcagacccag ctagcgctgg aggagaaggc ggtgctgcgg aaggaacgag acaacctggc 1140
caaggagctg gaagagaaga agagggaggc ggagcagctc aggatggagc tggccatcag 1200
aaactcagcc ctggacacct gcatcaagac caagtcgcag ccgatgatgc cagtgtcaag 1260
gcccatgggc cctgtcccca acccccagcc catcgaccca gctagcctgg aggagttcaa 1320
gaggaagatc ctggagtccc agaggccccc tgcaggcatc cctgtagccc catccagtgg 1380
ctgaggaggc tccaggcctg aggaccaagg gatggcccga ctcggcggtt tgcggaggat 1440
gcaggatatg ctcacagcgc cccgacacaa ccccctcccg ccgcccccaa ccacccaggg 1500
ccaccatcag acaactccct gcatgcaaac ccctagtacc ctctcacacc cgcacccgcg 1560
cctcacgatc cctcacccag agcacacggc cgcggagatg acgtcacgca agcaacggcg 1620
ctgacgtcac atatcaccgt ggtgatggcg tcacgtggcc atgtagacgt cacgaagaga 1680
tatagcgatg gcgtcgtgca gatgcagcac gtcgcacaca gacatgggga acttggcatg 1740
acgtcacacc gagatgcagc aacgacgtca cgggccatgt cgacgtcaca catattaatg 1800
tcacacagac gcggcgatgg catcacacag acggtgatga tgtcacacac agacacagtg 1860
acaacacaca ccatgacaac gacacctata gatatggcac caacatcaca tgcacgcatg 1920
ccctttcaca cacactttct acccaattct cacctagtgt cacgttcccc cgaccctggc 1980
acacgggcca aggtacccac aggatcccat cccctcccgc acagccctgg gccccagcac 2040
ctcccctcct ccagcttcct ggcctcccag ccacttcctc acccccagtg cctggacccg 2100
gaggtgagaa caggaagcca ttcacctccg ctccttgagc gtgagtgttt ccaggacccc 2160
ctcggggccc tgagccgggg gtgaggtcca cctgttgtcg ggagggggagc cactccttct 2220
cccccaactc ccagccctgc ctgtggcccg ttgaaatgtt ggtggcactt aataaatatt 2280
agtaaatcct taaaaaaaaa aaaaaaaaaa aaa

<210> 7
<211> 389
<212> DNA
<213> Human

<400> 7
   gccaaaaaga tggcttcaaa agtaagaatg aaacatttga tccattcagc tttaggctat 60
   gccactggat tcatgtctag aaaagatagg ataatttctg taaagaaatg aagaccttgc 120
   tattctaaaa tcagatcctt acagatccag atttcaggaa acaaatacat aggggactaa 180
   ctttccttgt tcagattagt ttttctcctt tgcacccagc tatataatat gaggaagtat 240
   tgacttttta aaagtgtttt agttttccat ttctttgata tgaaaagtaa tatttcggga 300
   gaaccctgag ctattaataa tctatgtggc tagtgcgtat atattggtct gaatttgttc 360
   tccttttgtg gtgtccagtg ggtaacatc

<210> 8
<211> 157
<212> DNA
<213> Human

<400> 8

tgctttaaac agctgtgtca aaaactgaca tcagagagta aattgaattt ggttttgtag 60
gaagcaggaa gcaagcccac tcaaacgtga aatttggcat gagggatcca gtaactttct 120
cctcaatctg tgaactatat gtgagtttga tattttg

<210> 9
<211> 561
<212> DNA
<213> Human

<400> 9

aatagtcaaa acataaacaa aagctaatta actggcactg ttgtcacctg agactaagtg 60
gatgttgttg gctgacatac aggctcagcc agcagagaaa gaattctgaa ttccccttgc 120
tgaactgaac tattctgtta catatggttg acaaatctgt gtgttatttc ttttctacct 180
accatattta aatttatgag tatcaaccga ggacatagtc aaaccttcga tgatgaacat 240
tcctgatttt ttgcctgatt aatctctgtt gagctctact tgtggtcatt caagatttta 300
tgatgttgaa aggaaaagtg aatatgacct ttaaaaattg tattttgggt gatgatagtc 360
tcaccactat aaaactgtca attattgcct aatgttaaag atatccatca ttgtgattaa 420
ttaaacctat aatgagtatt cttaatggag aattcttaat ggatggatta tcccctgatc 480
ttttctttaa aatttctctg cacacacagg acttctcatt ttccaataaa tgggtgtact 540
ctgccccaat ttctaggaaa a

<210> 10
<211> 1508
<212> DNA
<213> Human

<400> 10

cacaaacacg agagactcca cggtctgcct gagcaccgcc agcctcctag gctccagcac 60
tcgcaggtcc attcttctgc acgagcctct ctgtccagat ccataagcac ggtcagctca 120
gggtcgcgga gcagtacgag gacaagtacc agcagcagct cctctgaaca gagactgcta 180
ggatcatcct tctcctccgg gcctgttgct gatggcataa tccgggtgca acccaaatct 240
gagctcaagc caggtgagct taagccactg agcaaggaag atttgggcct gcacgcctac 300
aggtgtgagg actgtggcaa gtgcaaatgt aaggagtgca cctacccaag gcctctgcca 360
tcagactgga tctgcgacaa gcagtgcctt tgctcggccc agaacgtgat tgactatggg 420
acttgtgtat gctgtgtgaa aggtctcttc tatcactgtt ctaatgatga tgaggacaac 480
tgtgctgaca acccatgttc ttgcagccag tctcactgtt gtacacgatg gtcagccatg 540
ggtgtcatgt ccctcttttt gccttgttta tggtgttacc ttccagccaa gggttgcctt 600
aaattgtgcc aggggtgtta tgaccgggtt aacaggcctg gttgccgctg taaaaactca 660
aacacagttt gctgcaaagt tcccactgtc ccccctagga actttgaaaa accaacatag 720
catcattaat caggaatatt acagtaatga ggattttttc tttctttttt taatacacat 780
atgcaaccaa ctaaacagtt ataatcttgg cactgttaat agaaagttgg gatagtcttt 840
gctgtttgcg gtgaaatgct ttttgtccat gtgccgtttt aactgatatg cttgttagaa 900
ctcagctaat ggagctcaaa gtatgagata cagaacttgg tgacccatgt attgcataag 960
ctaaagcaac acagacactc ctaggcaaag tttttgtttg tgaatagtac ttgcaaaact 1020
tgtaaattag cagatgactt ttttccattg ttttctccag agagaatgtg ctatattttt 1080
gtatatacaa taatatttgc aactgtgaaa aacaagtggt gccatactac atggcacaga 1140
cacaaaatat tatactaata tgttgtacat tcggaagaat gtgaatcaat cagtatgttt 1200
ttagattgta ttttgcctta cagaaagcct ttattgtaag actctgattt ccctttggac 1260
ttcatgtata ttgtacagtt acagtaaaat tcaaccttta ttttctaatt ttttcaacat 1320

attgtttagt gtaaagaata tttatttgaa gttttattat tttataaaaa agaatattta 1380
ttttaagagg catcttacaa attttgcccc ttttatgagg atgtgatagt tgctgcaaat 1440
gaggggttac agatgcatat gtccaatata aaatagaaaa tatattaacg tttgaaatta 1500
aaaaaaaa

<210> 11
<211> 389
<212> DNA
<213> Human

<400> 11

gggcaggtga tcagggcaca catttcccgt ccattgagac agtagcattc ccggcaccca 60
tcgtgccagc tctcctcatt tttatgatga tgaccatcca cggtgagaca agtgcccgac 120
aggatgggtg gcccagctga agcacaggcc gctctgcact tgcagataag acagccgtga 180
ctgtcctgct ggaaacccaa ggggcagatc ttactgcatg agagctctgg acatttctta 240
cagcgacaga tgtcacagcc gtgcttattc ttcagcaatc caagtggaca atacttgtca 300
cagattatgg gtctgcactt cttgggcctt gggcggcact cacagatctc acagttttgg 360
acctcggccg cgaccacgct gggtaccga

<210> 12
<211> 981
<212> DNA
<213> Human

<400> 12

tttttttttt ttggattgca aaaatttatt aaaattggag acactgtttt aatcttcttg 60
tgccatgaga ctccatcagg cagtctacaa agaccactgg gaggctgagg atcacttgag 120
cccagaagtt tgaggctgta gtaagcttca aaggccactg cactctagct tgggtgaggc 180
aagacccttt caagcagtaa gctgcatgct tgcttgttgt ggtcattaaa aaccctagtt 240
taggataaca acatattaat cagggcaaaa tacaaatgtg tgatgcttgt tagtagagta 300
acctcagaat caaaatggaa cggttttaca gtgatatcat tatatttcat ttggcagaat 360
cattacatca ttggttacac tgaaaatcat cacatgtacc aaaagctgac tcacctagtt 420
taggataaca ggtctgcctg tttgaagatg aaaaataata cccatttaaa atttgcccta 480
ctcaatttcc ttctcagtca cattttaact tttaaacagc taatcactcc catctacaga 540
ttaaggtgta tatgccacca aaaccttttg ccaccttaaa aatttccttc aaagtttaaa 600
ctaatgcctg catttcttca atcatgaatt ctgagtcctt tgcttcttta aaacttgctc 660
cacacagtgt agtcaagccg actctccata cccaagcaag tcatccatgg ataaaaacgt 720
taccaggagc agaaccatta agctggtcca ggcaagttgg actccaccat ttcaacttcc 780
agctttctgt ctaatgcctg tgtgccaatg gcttgagtta ggcttgctct ttaggacttc 840
agtagctatt ctcatccttc cttggggaca caactgtcca taaggtgcta tccagagcca 900
cactgcatct gcacccagca ccatacctca caggagtcga ctcccacgag ccgcctgtat 960
ataagagttc ttttgatgac g

<210> 13
<211> 401
<212> DNA
<213> Human

<400> 13

ataactacag cttcagcaga caactaaaga gactgcatta aggtgatttc tctggctata 60
aagagagccc ggccgcagag catgtgactg ctgggacctc tgggataggc aacactgccc 120
tctctccccc agagcgaccc cccgggcagg tcggggccca aggaatgacc cagcaactgc 180
tccctaccca gcacactctc tttactgcca cctgcaatta tgctgtgaag atgactgggt 240
gtggtcatca cgattcagag aaatcaagat ctatgaccat tttaggcaaa gagagaaact 300
tggagaattg ctgaggacta ctgaaccttg ttttgctttt ttaaaaaata ctaaatcctc 360
acttcagcat atttagttgt cattaaaatt aagctgatat t

<210> 14
<211> 1002
<212> DNA
<213> Human

<400> 14

gacaatataa aaagtggaaa caagcataaa ttgcagacat aaaataatct tctggtagaa 60
acagttgtgg agaacaggtt gagtagagca acaacaacaa aagcttatgc agtcaccttc 120
tttgaaaatg ttaaatacaa gtcctattct ctttgtccag ctgggtttag ctagaggtag 180
ccaattactt ctcttaaggt ccatggcatt cgccaggatt ctataaaagc caagttaact 240
gaagtaaata tctggggccc atcgcacccc cactaagtac tttgtcacca tgttgtatct 300
taaaagtcat ttttcactgt ttgactcaga atttgggact tcagagtcaa acttcattgc 360
ttactccaaa cccagtttaa ttccccactt ttttaagtag gcttagcttt gagtgatttt 420
tggctataac cgaaatgtaa atccaccttc aaacaacaaa gtttgacaag actgaaatgt 480
tactgaaaac aatggtgcca tatgctccaa agacatttcc ccaagataac tgccaaagag 540
tttttgagga ggacaatgat catttattat gtaggagcct tgatatctct gcaaaataga 600
attaatacag ctcaaatgga gtagtaacca agcttttctg cccaggaagt aacaaacatc 660
actacgaaca tgagagtaca agaggaaact ttcataatgc attttttcat tcatacattc 720
attcaataaa cattagccaa gctaatgtcc caagccactg tgccaggtat taacaatata 780
acaacaataa aagacacagt ccttcctctc aaggtgttca gtctagtagg gaagatgatt 840
attcattaaa atttttggtg catcagaatc atgaggagct tgtcaaaaat gtaaattcct 900
gcctatgttc tcagatattc tggttaggtc aggagtggga acccaaaatc aattctttta 960
acaaacacta aaggtgattc taacacaggc ggtgtgagga cc

<210> 15
<211> 280
<212> DNA
<213> Human

<400> 15

cgaggtgggc cacccgtgtc tggtctgaga tttttaaatg aggattacat tatcctattt 60
ataatattcc tattctaatc tattgtattc ttacaattaa atgtatcaaa taattcttaa 120
aaacattatt agaaacaaac tgcctaatac cttataagac taaaaaaatc accaagatga 180
aactgtatta tgactctcaa tatttaaaca tttaaaaaaa tgttagtgtt tgttaagcac 240
caatcttaac tatttcacct gcccgggcgg ccgctcgagg

<210> 16
<211> 2041
<212> DNA
<213> Human

<400> 16

ccccccgcag aactcccccc tggaatagga tttttaaaac ccttgacaat tagaaatcct 60
atagaggtta gcatttttta ggtaaaaata tggttgcccc tacagggatc atgcaacttc 120
cttaaaacca attcagcaca tatgtataaa gaaccctttt taaaaacatt tgtacttgaa 180
atacagacac agtgatgctg aagacactaa acaaaaactg aaaagtacta taccttgata 240
aattttgtta ttgccttctt tagagacttt ataatctcta gttgattttc aaggacttga 300
atttaataat ggggtaatta cacaagacgt aaaggatttt ttaaaaacaa gtattttttt 360
ttacctctag catcaattct tttataaaga atgctaaata aattacattt tttgttcagt 420
aaaactgaag atagaccatt taaatgcttc taccaaattt aacgcagctt aattagggac 480
caggtacata ttttcttctg aacatttttg gtcaagcatg tctaaccata aaagcaaatg 540
gaattttaag aggtagattt tttttccatg atgcattttg ttaataaatg tgtcaagaaa 600
ataaaaacaa gcactgagtg tgttctcttg aagtataagg gtctaatgaa aaataaaaga 660
tagatatttg ttatagtctg acattttaac agtcatagta ttagacgttt cgtgaccagt 720
gcattttgga ctctctcagg atcaaaatac gagtctgcca actgtattaa atcctcctcc 780
accccctcca ccagttggtc cacagcttcc tggtgggtcg ttgtcatcaa atccattggg 840
ccgaaatgaa catgaagcag atgcagcttg gagggcccgg gctcgagcat tcaactcttg 900
ttcctgtaaa tatagtttat tgtcttttgt tatagcatcc ataagttctt tctgtagagg 960
tgggtctcca tttatccaga gtccactggt tgggttatta ccacttaaac cattagtact 1020
atgctgtttt ttatacaaaa gcacataagc tgtgtccttt ggaaacctgc tcgtaatttt 1080
ctggactgac tgaaatgaag taaatgtcac tctactgtca ttaaataaaa acccattctt 1140
ttgacatttc cttattttcc aaatcctgtt caaaaactgc actgggacta tctctcccta 1200
gtaaatgact ctgggaggat gctaatgcca gagcctcaga ctggtggtac atctgatatg 1260
aagagtctgt acttgtgata tttctggcat aagaatagta atgcccactt tcagaggata 1320
taccagagtg aaccacaacg gaacttaata gatagggcac caattttgtg caggaagctt 1380
catcagtccc tgaaggcttt aattttttag caaggttctc actaagatca gtgaagtcaa 1440
catctacaga ccaactttct gacaatgaag agaaagaagt aattcttcta actggcaact 1500
ccaaaaccag tggccagtga tacattgtct aaaattttcc ttctcacatg atacttctga 1560
tcatatgaaa atctcaggag agtaagaata aggtattcag gttcctccgt gatttgcata 1620

gttttctcag cattttgcag agaggcacag ttttcacaat aatattggtt atcaccagta 1680
agaatctctg gagcccaaaa aataatttag taagtcagtt actgaaggtg tggtttcacc 1740
tcccggtttc tgaggtacat ctttattaac aagaatcttg ttagattcgt tagggacaga 1800
agtgttttca gaacagtaaa actcattagg aggactgcct atggtttttt cattcacaag 1860
tgagtcacag atgaaggcag ctgttgttgg attataaact actggctctt ctgaaggacc 1920
gggtacagac gcttgcatta gaccaccatc ttgtatactg ggtgatgatg ctggatcttg 1980
gacagacatg ttttccaaag aagaggaagc acaaaacgca agcgaaagat ctgtaaaggc 2040
t

<210> 17
<211> 235
<212> DNA
<213> Human

<400> 17

cgccccgggc aggtgtcagg ggttccaaac cagcctgggg aaacacagcg tagacccctc 60
acctctacaa ataaaaaatt aaaaaattag ccaggtgtgg cagcgaacaa ctgtagtctc 120
agatactcag gagactgagc tggaaaggat cacttgagcc caagaagttc aaggttacag 180
tgggccacga tcatgtcatt acactccagc ttgggtgaca aaatgagact gtcta

<210> 18
<211> 2732
<212> DNA
<213> Human

<400> 18

gtgtggagtt tcagctgcta ttgactataa gagctatgga acagaaaaag cttgctggct 60
tcatgttgat aactacttta tatggagctt cattggacct gttaccttca ttattctgct 120
aaatattatc ttcttggtga tcacattgtg caaaatggtg aagcattcaa acactttgaa 180
accagattct agcaggttgg aaaacattaa gtcttgggtg cttggcgctt tcgctcttct 240
gtgtcttctt ggcctcacct ggtcctttgg gttgcttttt attaatgagg agactattgt 300
gatggcatat ctcttcacta tatttaatgc tttccaggga gtgttcattt tcatctttca 360
ctgtgctctc caaaagaaag tacgaaaaga atatggcaag tgcttcagac actcatactg 420
ctgtggaggc ctcccaactg agagtcccca cagttcagtg aaggcatcaa ccaccagaac 480
cagtgctcgc tattcctctg gcacacagag tcgtataaga agaatgtgga atgatactgt 540
gagaaaacaa tcagaatctt cttttatctc aggtgacatc aatagcactt caacacttaa 600
tcaaggtggc ataaatctta atatattatt acaggactga catcacatgg tctgagagcc 660
catcttcaag atttatatca tttagaggac attcactgaa caatgccagg gatacaagtg 720
ccatggatac tctaccgcta aatggtaatt ttaacaacag ctactcgctg cacaagggtg 780
actataatga cagcgtgcaa gttgtggact gtggactaag tctgaatgat actgcttttg 840
agaaaatgat catttcagaa ttagtgcaca acaacttacg gggcagcagc aagactcaca 900
acctcgagct cacgctacca gtcaaacctg tgattggagg tagcagcagt gaagatgatg 960
ctattgtggc agatgcttca tctttaatgc acagcgacaa cccagggctg gagctccatc 1020
acaaagaact cgaggcacca cttattcctc agcggactca ctcccttctg taccaacccc 1080
agaagaaagt gaagtccgag ggaactgaca gctatgtctc ccaactgaca gcagaggctg 1140
aagatcacct acagtccccc aacagagact ctctttatac aagcatgccc aatcttagag 1200
actctcccta tccggagagc agccctgaca tggaagaaga cctctctccc tccaggagga 1260
gtgagaatga ggacatttac tataaaagca tgccaaatct tggagctggc catcagcttc 1320
agatgtgcta ccagatcagc aggggcaata gtgatggtta tataatcccc attaacaaag 1380
aagggtgtat tccagaagga gatgttagag aaggacaaat gcagctggtt acaagtcttt 1440
aatcatacag ctaaggaatt ccaagggcca catgcgagta ttaataaata aagacaccat 1500
tggcctgacg cagctccctc aaactctgct tgaagagatg actcttgacc tgtggttctc 1560
tggtgtaaaa aagatgactg aaccttgcag ttctgtgaat ttttataaaa catacaaaaa 1620
ctttgtatat acacagagta tactaaagtg aattatttgt tacaaagaaa agagatgcca 1680
gccaggtatt ttaagattct gctgctgttt agagaaattg tgaaacaagc aaaacaaaac 1740
tttccagcca ttttactgca gcagtctgtg aactaaattt gtaaatatgg ctgcaccatt 1800
tttgtaggcc tgcattgtat tatatacaag acgtaggctt taaaatcctg tgggacaaat 1860
ttactgtacc ttactattcc tgacaagact tggaaaagca ggagagatat tctgcatcag 1920
tttgcagttc actgcaaatc ttttacatta aggcaaagat tgaaaacatg cttaaccact 1980
agcaatcaag ccacaggcct tatttcatat gtttcctcaa ctgtacaatg aactattctc 2040
atgaaaaatg gctaaagaaa ttatattttg ttctattgct agggtaaaat aaatacattt 2100
gtgtccaact gaaatataat tgtcattaaa ataattttaa agagtgaaga aaatattgtg 2160
aaaagctctt ggttgcacat gttatgaaat gttttttctt acactttgtc atggtaagtt 2220
ctactcattt tcacttcttt tccactgtat acagtgttct gctttgacaa agttagtctt 2280

tattacttac atttaaattt cttattgcca aaagaacgtg ttttatgggg agaaacaaac 2340
tctttgaagc cagttatgtc atgccttgca caaaagtgat gaaatctaga aaagattgtg 2400
tgtcacccct gtttattctt gaacagaggg caaagagggc actgggcact tctcacaaac 2460
tttctagtga acaaaaggtg cctattcttt tttaaaaaaa taaaataaaa cataaatatt 2520
actcttccat attccttctg cctatattta gtaattaatt tattttatga taaagttcta 2580
atgaaatgta aattgtttca gcaaaattct gctttttttt catccctttg tgtaaacctg 2640
ttaataatga gcccatcact aatatccagt gtaaagttta acacggtttg acagtaaata 2700
aatgtgaatt ttttcaagtt aaaaaaaaaa aa

<210> 19
<211> 276
<212> DNA
<213> Human

<400> 19

ctccctaaat gattttaaaa taaattggat aaacatatga tataaagtgg gtactttaga 60
aaccgccttt gcatattttt tatgtacaaa tctttgtata caattccgat gttccttata 120
tattccctat atagcaaacc aaaaccagga cctcccaact gcatgcctca agtccctgtg 180
gagcactctg gcaactggat ggccctactt gctttctgac aaaatagctg gaaaggagga 240
gggaccaatt aaatacctcg gccgcgacca cgctgg

<210> 20
<211> 2361
<212> DNA
<213> Human

<400> 20

attgtaccag ccttgatgaa cgtgggccct gcttcgcttt tgagggccat aagctcattg 60
cccactggtt tagaggctac cttatcattg tctcccgtga ccggaaggtt tctcccaagt 120
cagagtttac cagcagggat tcacagagct ccgacaagca gattctaaac atctatgacc 180
tgtgcaacaa gttcatagcc tatagcaccg tctttgagga tgtagtggat gtgcttgctg 240
agtggggctc cctgtacgtg ctgacgcggg atgggcgggt ccacgcactg caggagaagg 300
acacacagac caaactggag atgctgttta agaagaacct atttgagatg gcgattaacc 360
ttgccaagag ccagcatctg gacagtgatg ggctggccca gattttcatg cagtatggag 420
accatctcta cagcaagggc aaccacgatg gggctgtcca gcaatatatc cgaaccattg 480
gaaagttgga gccatcctac gtgatccgca agtttctgga tgcccagcgc attcacaacc 540
tgactgccta cctgcagacc ctgcaccgac aatccctggc caatgccgac cataccaccc 600
tgctcctcaa ctgctatacc aagctcaagg acagctcgaa gctggaggag ttcatcaaga 660
aaaagagtga gagtgaagtc cactttgatg tggagacagc catcaaggtc ctccggcagg 720
ctggctacta ctcccatgcc ctgtatctgg cggagaacca tgcacatcat gagtggtacc 780
tgaagatcca gctagaagac attaagaatt atcaggaagc ccttcgatac atcggcaagc 840
tgccttttga gcaggcagag agcaacatga agcgctacgg caagatcctc atgcaccaca 900
taccagagca gacaactcag ttgctgaagg gactttgtac tgattatcgg cccagcctcg 960
aaggccgcag cgatagggag gccccaggct gcagggccaa ctctgaggag ttcatcccca 1020
tctttgccaa taacccgcga gagctgaaag ccttcctaga gcacatgagt gaagtgcagc 1080
cagactcacc ccaggggatc tacgacacac tccttgagct gcgactgcag aactgggccc 1140
acgagaagga tccacaggtc aaagagaagc ttcacgcaga ggccatttcc ctgctgaaga 1200
gtggtcgctt ctgcgacgtc tttgacaagg ccctggtcct gtgccagatg cacgacttcc 1260
aggatggtgt cctttacctt tatgagcagg ggaagctgtt ccagcagatc atgcactacc 1320
acatgcagca cgagcagtac cggcaggtca tcagcgtgtg tgagcgccat ggggagcagg 1380
acccctcctt gtgggagcag gccctcagct acttcgctcg caaggaggag gactgcaagg 1440
agtatgtggc agctgtcctc aagcatatcg agaacaagaa cctcatgcca cctcttctag 1500
tggtgcagac cctggcccac aactccacag ccacactctc cgtcatcagg gactacctgg 1560
tccaaaaact acagaaacag agccagcaga ttgcacagga tgagctgcgg gtgcggcggt 1620
accgagagga gaccacccgt atccgccagg agatccaaga gctcaaggcc agtcctaaga 1680
ttttccaaaa gaccaagtgc agcatctgta acagtgcctt ggagttgccc tcagtccact 1740
tcctgtgtgg ccactccttc caccaacact gctttgagag ttactcggaa agtgatgctg 1800
actgccccac ctgcctccct gaaaaccgga aggtcatgga tatgatccgg gcccaggaac 1860
agaaacgaga tctccatgat caattccagc atcagctcaa gtgctccaat gacagctttt 1920
ctgtgattgc tgactacttt ggcagaggtg ttttcaacaa attgactctg ctgaccgacc 1980
ctcccacagc cagactgacc tccagcctgg aggctgggct gcaacgcgac ctactcatgc 2040
actccaggag gggcacttaa gcagcctgga ggaagatgtg ggcaacagtg gaggaccaag 2100
agaacagaca caatgggacc tgggcgggcg ttacacagaa ggctggctga catgcccagg 2160
gctccactct catctaatgt cacagccctc acaagactaa agcggaactt tttcttttcc 2220

ctggccttcc ttaattttaa gtcaagcttg gcaatccctt cctctttaac taggcaggtg 2280
ttagaatcat ttccagatta atggggggga aggggaacct caggcaaacc tcctgaagtt 2340
ttggaaaaaa aagctggttt c

<210> 21
<211> 179
<212> DNA
<213> Human

<400> 21

aggtgttaga tgctcttgaa aaagaaactg catctaagct gtcagaaatg gattctttta 60
acaatcaact aaaggaactg agagaaacct acaacacaca gcagttagcc cttgaacagc 120
tttataagat caacgtgaca agttgaagga aattgaaagg aaaaaattag aactaatgc

<210> 22
<211> 905
<212> DNA
<213> Human

<400> 22

tttttttttt ttctttaacc gtgtggtctt tatttcagtg ccagtgttac agatacaaca 60
caaatgttcc agttagaagg aattcaaacg gaatgccaag gtccaagcca ggctcaagaa 120
ataaaaaggg aggtttggag taatagataa gatgactcca atactcactc ttcctaaggg 180
caaaggtact tttgatacag agtctgatct ttgaaactgg tgaactcctc ttccacccat 240
taccatagtt caaacaggca agttatgggc ttaggagcac tttaaaattt gtggtgggaa 300
tagggtcatt aataactatg aatatatctt ttagaaggtg accattttgc actttaaagg 360
gaatcaattt tgaaaatcat ggagactatt catgactaca gctaaagaat ggcgagaaag 420
gggagctgga agagccttgg aagtttctat tacaaataga gcaccatatc cttcatgcca 480
aatctcaaca aaagctcttt ttaactccat ctgtccagtg tttacaaata aactcgcaag 540
gtctgaccag ttcttggtaa caaacataca tgtgtgtgtc tgtgtgtata cagcaatgca 600
cagaaaaggc taccaggagc ctaatgcctc tttcaaacat tgggggaacc agtagaaaaa 660
ggcagggctc cctaatgtcc attattacat ttccattccg aatgccagat gttaaaagtg 720
cctgaagatg gtaacccagc tagtgaggaa taaatacccc accttgccca gtccacagag 780
aaacaacagt agaaagaagg ggcaactctt tgctgcagag acaaagtgag tgttttttcg 840
ccatggattg cagtcctctc ctccagacca gctgcttatt tcctcagggg cccagggaat 900
gttga

<210> 23
<211> 2134
<212> DNA
<213> Human

<400> 23

ggtctcttct ttcctttttt tttttccaaa agtgttcttt tatttctagt aacatatatt 60
gtataaatac tctattttat atgcacttcc acaaaagcga tataatttaa aagttttttt 120
cattagaaat aaatgtataa aaataaatat gttattatag gcatttatta ctaactatag 180
tccttcttgg aaggaacacc caaaccaata cttataaagt acatgtaatt tatagtaaca 240
tattttacta tatacatatg gaaaaaatca tattctcaca gaagagctga acagacattc 300
accaggatac gactgttgga ccagctgctg gagatggacc tgctacccct cagcagcctc 360
cccaccacaa gacaagtgat ctcaatgtcc ccaaacctgt gggaccctgt tctacacacc 420
tcatttttgt tccggcgttt catcctcctt gtgtgattgt actgattttc atgagacaca 480
agttacttct ttacatccat attcccaaag cagggttaca tggtaggaaa gaaaggaagt 540
tggaggtact aagctcattg tgtctcctct agcttttacc agcatctaat gcttcactgc 600
tttttttcca ttgtagactt taatgcactt gaataaatac atggagttgt tttttcctca 660
aaatgaatta cacaaataaa gactgagatg gtccaaaaaa ggaaagagga agccatttgc 720
gttatttcac gttgctgagc ctttctctca tgttgaacaa tctgaagttt taattctcgg 780
tagaaataat gtataaacat tctctgaaac catagcagcc ataaacagtg ctggtcaaag 840
atcctatttg tactcctttc tccccccatt gttagtgagg taaagtaaaa caggtcttag 900
taaaatctca cttttctcct acttttcatt tcccaacccc catgatacta agtatttgat 960
aagtaccagg aaacaggggt tgtaatagtt ctaacttttt ttgacaattg ctttgttttt 1020
tctaaacttg taatagatgt aacaaaagaa ataataataa taatgcccgg ggctttatta 1080
tgctatatca ctgctcagag gttaataatc ctcactaact atcctatcaa atttgcaact 1140
ggcagtttac tctgatgatt caactccttt tctatctacc cccataatcc caccttactg 1200

atacacctca ctggttactg gcaagatacg ctggatccct ccagccttct tgctttccct 1260
gcaccagccc ttcctcactt tgccttgccc tcaaagctaa caccacttaa accacttaac 1320
tgcattctgc cattgtgcaa aagtctatga aatgtttagg tttctttaaa ggatcacagc 1380
tctcatgaga taacacccct ccatcatggg acagacactt caagcttctt tttttgtaac 1440
ccttcccaca ggtcttagaa catgatgacc actcccccag ctgccactgg gggcagggat 1500
ggtctgcaca aggtctggtg ctggctggct tcacttcctt tgcacactcg gaagcaggct 1560
gtccattaat gtctcggcat tctaccagtc ttctctgcca acccaattca catgacttag 1620
aacattcgcc ccactcttca atgacccatg ctgaaaaagt ggggatagca ttgaaagatt 1680
ccttcttctt ctttacgaag taggtgtatt taattttagg tcgaagggca ttgcccacag 1740
taagaacctg gatggtcaag ggctctttga gagggctaaa gctgcgaatt ctttccaatg 1800
ccgcagagga gccgctgtac ctcaagacaa cacctttgta cataatgtct tgctctaagg 1860
tggacaaagt gtagtcacca ttaagaatat atgtgccatc agcagctttg atggcaagaa 1920
agctgccatt gttcctggat cccctctggt tccgctgttt cacttcgatg ttggtggctc 1980
cagttggaat tgtgatgata tcatgatatc caggttttgc actagtaact gatcctgata 2040
tttttttaca agtagatcca tttcccccgc aaacaccaca tttatcaaac ttctttttgg 2100
agtctatgat gcgatcacaa ccagctttta caca

<210> 24
<211> 1626
<212> DNA
<213> Human

<400> 24

ggacaatttc tagaatctat agtagtatca ggatatattt tgctttaaaa tatattttgg 60
ttattttgaa tacagacatt ggctccaaat tttcatcttt gcacaatagt atgacttttc 120
actagaactt ctcaacattt gggaactttg caaatatgag catcatatgt gttaaggctg 180
tatcatttaa tgctatgaga tacattgttt tctccctatg ccaaacaggt gaacaaacgt 240
agttgttttt tactgatact aaatgttggc tacctgtgat tttatagtat gcacatgtca 300
gaaaaaggca agacaaatgg cctcttgtac tgaatacttc ggcaaactta ttgggtcttc 360
attttctgac agacaggatt tgactcaata tttgtagagc ttgcgtagaa tggattacat 420
ggtagtgatg cactggtaga aatggttttt agttattgac tcagaattca tctcaggatg 480
aatcttttat gtctttttat tgtaagcata tctgaattta ctttataaag atggttttag 540
aaagctttgt ctaaaaattt ggcctaggaa tggtaacttc attttcagtt gccaaggggt 600
agaaaaataa tatgtgtgtt gttatgttta tgttaacata ttattaggta ctatctatga 660
atgtatttaa atatttttca tattctgtga caagcattta taatttgcaa caagtggagt 720
ccatttagcc cagtgggaaa gtcttggaac tcaggttacc cttgaaggat atgctggcag 780
ccatctcttt gatctgtgct taaactgtaa tttatagacc agctaaatcc ctaacttgga 840
tctggaatgc attagttatg ccttgtacca ttcccagaat ttcaggggca tcgtgggttt 900
ggtctagtga ttgaaaacac aagaacagag agatccagct gaaaaagagt gatcctcaat 960
atcctaacta actggtcctc aactcaagca gagtttcttc actctggcac tgtgatcatg 1020
aaacttagta gaggggattg tgtgtatttt atacaaattt aatacaatgt cttacattga 1080
taaaattctt aaagagcaaa actgcatttt atttctgcat ccacattcca atcatattag 1140
aactaagata tttatctatg aagatataaa tggtgcagag agactttcat ctgtggattg 1200
cgttgtttct tagggttcct agcactgatg cctgcacaag catgtgatat gtgaaataaa 1260
atggattctt ctatagctaa atgagttccc tctggggaga gttctggtac tgcaatcaca 1320
atgccagatg gtgtttatgg gctatttgtg taagtaagtg gtaagatgct atgaagtaag 1380
tgtgtttgtt ttcatcttat ggaaactctt gatgcatgtg cttttgtatg gaataaattt 1440
tggtgcaata tgatgtcatt caactttgca ttgaattgaa ttttggttgt atttatatgt 1500
attatacctg tcacgcttct agttgcttca accattttat aaccattttt gtacatattt 1560
tacttgaaaa tattttaaat ggaaatttaa ataaacattt gatagtttac ataataaaaa 1620
aaaaaa

<210> 25
<211> 1420
<212> DNA
<213> Human

<400> 25

gttcagcatt gtttctgctt ctgaaatctg tatagtacac tggtttgtaa tcattatgtc 60
ttcattgaaa tccttgctac ttctcttcct cctcaatgaa agacacgaga gacaagagcg 120
acacaagctt aagaaaaacg agcaaggaag agtatcttca ttattctcat tttctctgag 180
ttggaaacaa aaacatgaag gactccaact agaagacaga tatttacatt taaatagatt 240
agtgggaaaa ctttaagagt ttccacatat tagttttcat tttttgagtc aagagactgc 300
tccttgtact gggagacact agtagtatat gtttgtaatg ttactttaaa attatctttt 360

tattttataa ggcccataaa tactggttaa actctgttaa aagtgggcct tctatcttgg 420
atggtttcac tgccatcagc catgctgata tattagaaat ggcatcccta tctacttact 480
ttaatgctta aaattataca taaaatgctt tatttagaaa acctacatga tacagtggtg 540
tcagccttgc catgtatcag tttcacttga aatttgagac caattaaatt tcaactgttt 600
agggtggaga aagaggtact ggaaaacatg cagatgagga tatcttttat gtgcaacagt 660
atcctttgca tgggaggaga gttactcttg aaaggcaggc agcttaagtg gacaatgttt 720
tgtatatagt tgagaatttt acgacacttt taaaaattgt gtaattgtta aatgtccagt 780
tttgctctgt tttgcctgaa gttttagtat ttgttttcta ggtggacctc tgaaaaccaa 840
accagtacct ggggaggtta gatgtgtgtt tcaggcttgg agtgtatgag tggttttgct 900
tgtattttcc tccagagatt ttgaacttta ataattgcgt gtgtgttttt ttttttttaa 960
gtggctttgt ttttttttct caagtaaaat tgtgaacata tttcctttat aggggcaggg 1020
catgagttag ggagactgaa gagtattgta gactgtacat gtgccttctt aatgtgtttc 1080
tcgacacatt ttttttcagt aacttgaaaa ttcaaaaggg acatttggtt aggttactgt 1140
acatcaatct atgcataaat ggcagcttgt tttcttgagc cactgtctaa attttgtttt 1200
tatagaaatt ttttatactg attggttcat agatggtcag ttttgtacac agactgaaca 1260
atacagcact ttgccaaaaa tgagtgtagc attgtttaaa cattgtgtgt taacacctgt 1320
tctttgtaat tgggttgtgg tgcattttgc actacctgga gttacagttt tcaatctgtc 1380
agtaaataaa gtgtccttta acttcaaaaa aaaaaaaaaa

<210> 26
<211> 689
<212> DNA
<213> Human

<400> 26

aaacaaacaa aaaaaaagtt agtactgtat atgtaaatac tagcttttca atgtgctata 60
caaacaatta tagcacatcc ttccttttac tctgtctcac ctcctttagg tgagtacttc 120
cttaaataag tgctaaacat acatatacgg aacttgaaag ctttggttag ccttgcctta 180
ggtaatcagc ctagtttaca ctgtttccag ggagtagttg aattactata aaccattagc 240
cacttgtctc tgcaccattt atcacaccag gacagggtct ctcaacctgg gcgctactgt 300
catttggggc caggtgattc ttccttgcaa gggctgtcct gtacctgccc gggcggccgc 360
tcgaagcgtg gtcgcggccg aggtactgaa aggaccaagg agctctggct gccctcagga 420
attccaaatg accgaaggaa caaagcttca gggctctggg tggtgtctcc cactattcag 480
gaggtggtcg gaggtaacgc agcttcattt cgtccagtcc tttccagtat ttaaagttgt 540
tgtcaagatg ctgcattaaa tcaggcaggt ctacaaaggc atcccaagca tcaaacatgt 600
ctgtgatgaa gtaatcaatg aaacaccgga acctccgacc acctcctgaa tagtgggaga 660
cacacccaga gcctgaagtt tgtccttcg

<210> 27
<211> 471
<212> DNA
<213> Human

<400> 27

tcccagcggc atgaagtttg agattggcca ggccctgtac ctgggcttca tctccttcgt 60
ccctctcgct cattggtggc accctgcttt gcctgtcctg ccaggacgag gcaccctaca 120
agccctaacc caggccccgc ccaggccacc cacgaccact gcaaacaccg cacctgccta 180
ccagccacca gctgcctaca aagacaatcg ggccccctca gtgacctcgg ccaccacagc 240
gggtacaggc tgaacgacta cgtgtgagtc cccacagcct gcttctcccc tgggctgctg 300
tgggctggtt cccggcggga ctgtcaatgg aggcaggggt tccagcacaa agtttacttc 360
tgggcaattt ttgtatccaa ggaaataatg tgaatgcgag gaaatgtctt tagagcacag 420
ggacagaggg ggaaataaga ggaggagaaa gctctctata ccaaagactg a

<210> 28
<211> 929
<212> DNA
<213> Human

<400> 28

ggtgaactca gtgcattggg ccaatggttc gacacaggct ctgccagcca caaccatcct 60
gctgcttctg acggtttggc tgctggtggg ctttcccctc actgtcattg gaggcatctt 120
tgggaagaac aacgccagcc cctttgatgc accctgtcgc accaagaaca tcgcccggga 180
gattccaccc cagccctggt acaagtctac tgtcatccac atgactgttg gaggcttcct 240

gcctttcagt gccatctctg tggagctgta ctacatcttt gccacagtat ggggtcggga 300
gcagtacact ttgtacggca tcctcttctt tgtcttcgcc atcctgctga gtgtgggggc 360
ttgcatctcc attgcactca cctacttcca gttgtctggg gaggattacc gctggtggtg 420
gcgatctgtg ctgagtgttg gctccaccgg cctcttcatc ttcctctact cagttttcta 480
ttatgcccgg cgctccaaca tgtctggggc agtacagaca gtagagttct tcggctactc 540
cttactcact ggttatgtct tcttcctcat gctgggcacc atctcctttt tttcttccct 600
aaagttcatc cggtatatct atgttaacct caagatggac tgagttctgt atggcagaac 660
tattgctgtt ctctcccttt cttcatgccc tgttgaactc tcctaccagc ttctcttctg 720
attgactgaa ttgtgtgatg gcattgttgc cttccctttt tccctttggg cattccttcc 780
ccagagaggg cctggaaatt ataaatctct atcacataag gattatatat ttgaactttt 840
taagttgcct ttagttttgg tcctgatttt tctttttaca attaccaaaa taaaatttat 900
taagaaaaag aaaaaaaaaa aaaaaaaaa

<210> 29
<211> 1775
<212> DNA
<213> Human

<400> 29

gaacgtgatg ggaactttgg gaggatgtct gagaaaatgt ccgaagggat tttggccaac 60
accagaaaac gccaatgtcc taggaattcc ctcccaaaat gcttcccaaa aaattactca 120
ttgacaattc aaattgcact tggctggcgg cagcccgggc ggccttcagt ccgtgtgggg 180
cgcccgcgtg gccttctcct cgtaggactc cccaaactcg ttcactctgc gtttatccac 240
aggataaagc caccgctggt acaggtagac cagaaacacc acgtcgtccc ggaagcaggc 300
cagccggtga gacgtgggca tggtgatgat gaaggcaaag acgtcatcaa tgaaggtgtt 360
gaaagccttg taggtgaagg ccttccaggg cagatgtgcc actgacttca acttgtagtt 420
cacaaagagc tggggcagca tgaagaggaa accaaaggca tagaccccgt tgacgaagct 480
gttgattaac caggagtacc agctcttata tttgatattc aggagtgaat agacagcacc 540
cccgacacag agagggtaca gcaggtatga caagtacttc atggcctgag tatcgtactc 600
ctcggttttc ctctcagatt cgctgtaagt gccaaactga aattcgggca tcaggcctct 660
ccaaaaaata gtcatcttca atgccttctt cactttccac agctcaatgg cggctccaac 720
acccgccggg accagcacca gcaggctcgt ctgctcgtcc agcaggaaca gaaagatgac 780
cacggtgctg aagcagcgcc agagcactgc cttggtggac atgccgatca tgctcttctt 840
cttcttccag aaactgatgt catttttaaa ggccaggaaa tcaaagagaa gatggaacgc 900
tgcgacaaag aaggtcagcg ccaggaagta taagttggta tctacaaaaa ttcctttcac 960
ctcatcagca tctttctctg aaaacccgaa ctgctgcagg gagtacacgg cgtcctgcat 1020
gtggatccag aagcgcagcc gccccagtga gaccttgtcg taggacacgg tgaggggcag 1080
ctcggtggtg gagcggttta tgaccatcag gtccttcacg cggttgctga gctggtcgat 1140
gaacaggatg ggcaggtaat gcacggtttt ccccagctgg atcatcttca tgtaccgatg 1200
cacatcggca ggcagggagg acccgtcaaa gacaaagttg tccgccatca cgttcagcgc 1260
cagccgcggt cgccagtggg acactggctc atccagggca ctcgtcggct tcttctccgc 1320
ctcgatctgc tgtgtatcag actccccggt gagcaggttg atttcttctg gcttggggac 1380
catgtaggtg gtcagaggac tgaccaggtg cacctgcttc ccgtcgtgcc acggcaggac 1440
cccagcgtga tggaggaaga tgtaggcata cagcgtccca ttgtttctcg ttttctttgg 1500
tacagaaaca ttaactgtcc tttcaaattt ggactccaca tcaaagtctt ccacattcaa 1560
gaccaggtcg atgttgttct cagcacccag gtgggacctc gtcgtggtgt acacgctcag 1620
ctgcagcttg ggccgccgcg ccaggtaggg ctggatgcag ttggcgtcgc cggagcacgg 1680
gcgggtgtag acgatgccgt acatgaccca gcaggtgtgc accacgtaga ccacgaacac 1740
gcccaccacc aagctggtga aggagctgcg gcccc

<210> 30
<211> 1546
<212> DNA
<213> Human

<400> 30

aaaataagta ggaatgggca gtgggtattc acattcacta caccttttcc atttgctaat 60

aaggccctgc caggctggga gggaattgtc cctgcctgct tctggagaaa gaagatattg 120
acaccatcta cgggcaccat ggaactgctt caagtgacca ttctttttct tctgcccagt 180
atttgcagca gtaacagcac aggtgtttta gaggcagcta ataattcact tgttgttact 240
acaacaaaac catctataac aacaccaaac acagaatcat tacagaaaaa tgttgtcaca 300
ccaacaactg gaacaactcc taaaggaaca atcaccaatg aattacttaa aatgtctctg 360
atgtcaacag ctactttttt aacaagtaaa gatgaaggat tgaaagccac aaccactgat 420

gtcaggaaga atgactccat catttcaaac gtaacagtaa caagtgttac acttccaaat 480
gctgtttcaa cattacaaag ttccaaaccc aagactgaaa ctcagagttc aattaaaaca 540
acagaaatac caggtagtgt tctacaacca gatgcatcac cttctaaaac tggtacatta 600
acctcaatac cagttacaat tccagaaaac acctcacagt ctcaagtaat aggcactgag 660
ggtggaaaaa atgcaagcac ttcagcaacc agccggtctt attccagtat tattttgccg 720
gtggttattg ctttgattgt aataacactt tcagtatttg ttctggtggg tttgtaccga 780
atgtgctgga aggcagatcc gggcacacca gaaaatggaa atgatcaacc tcagtctgat 840
aaagagagcg tgaagcttct taccgttaag acaatttctc atgagtctgg tgagcactct 900
gcacaaggaa aaaccaagaa ctgacagctt gaggaattct ctccacacct aggcaataat 960
tacgcttaat cttcagcttc tatgcaccaa gcgtggaaaa ggagaaagtc ctgcagaatc 1020
aatcccgact tccatacctg ctgctggact gtaccagacg tctgtcccag taaagtgatg 1080
tccagctgac atgcaataat ttgatggaat caaaaagaac cccggggctc tcctgttctc 1140
tcacatttaa aaattccatt actccattta caggagcgtt cctaggaaaa ggaattttag 1200
gaggagaatt tgtgagcagt gaatctgaca gcccaggagg tgggctcgct gataggcatg 1260
actttcctta atgtttaaag ttttccgggc caagaatttt tatccatgaa gactttccta 1320
cttttctcgg tgttcttata ttacctactg ttagtattta ttgtttacca ctatgttaat 1380
gcagggaaaa gttgcacgtg tattattaaa tattaggtag aaatcatacc atgctacttt 1440
gtacatataa gtattttatt cctgctttcg tgttactttt aataaataac tactgtactc 1500
aatactctaa aaatactata acatgactgt gaaaatggca aaaaaa

<210> 31
<211> 750
<212> DNA
<213> Human

<400> 31

cacttgggca cccccatttt ctaaaaaaat ggaaatctgg agggcaaaaa aggtgtgctg 60
aagggaagtg cctctgatgg cccaaaaacc ttcttccaaa ctagtgtagg aatggaatgg 120
atagcaaatg gatccttttt ggcctccttt ggagcatgcc ttccctatct tatccttggc 180
cccactaaag cagaacgtta cggatatttc tgtttttgcc attggatgcc tatctggcca 240
aacagccttt ccctaattgg aaaatgcagt cctgtttaaa acctttgatt tacgactact 300
tgtacatgct tgctcattac aattttgaca ttttttacat agtgaagacc ccaaacatat 360
cagtgaaaca tgacaagatc ataaagaaca gtatcatatt attatttagt cgcttttaca 420
gtggcaagcc aattttgaaa tatctcattt aaaactcaga cccaattcac tgagttatac 480
ttttaatagc ttcctcagca cactatttcc catgcattaa atatgataaa ataatctatc 540
actgcccatc ggtcttgtaa aaaggaagtc tgaatacaga gcccacaaca ctaaaattgt 600
ttttctagct acaaagtata gcatcatcaa cacagacacg atttggactc cctgacaggt 660
ggattggaaa acggtgttta aagagaagag aacattttaa cataaatgtc attaagaatc 720
ccaaaggcct tatttgtcac caccgtcccg

<210> 32
<211> 1620
<212> DNA
<213> Human

<400> 32

gcaattcccc cctcccacta aacgactccc agtaattatg tttacaaccc attggatgca 60
gtgcagccat tcataagaac cttggtgccc cagaaaaatc tgtccttttt ggtaccaaac 120
ctgaggtctt ttggaagata atgtagaaaa ccactaccta ttgaaggcct gttttggcta 180
atctgtgcaa actctgatga tacctgcctt atgtggattc ttttccacac tgctttcatt 240
tttaagtata aagacttaga aaactagaat aatgctttta caaataatta aaagtatgtg 300
atgttctggg ttttttcctt ctttttagaa ccccgcctcc atttaaaaaa ttaaaaaaaa 360
aaaaaaaact tttaacattt aaaaaataaa aattaacaaa atttcactta ttccaggaca 420
cgctggcatt tggactcaat gaaaagggca cctaaagaaa ataaggctga ctgaatgttt 480
tccataattt tcacacaata acagtccctt tctatccagc ttgccttcca tttatctcta 540
gggttagctt ttcaggcaac atccttggtc attgcccaga aagtacctga gctatcagtg 600
attggaatgg cacaggaaac cgaatcacat gggtgccctc cccttggttt tcaagtatct 660
tggagttgtg cacaaaaatt aggtcatgcc ttcagtgtct tgttctttaa acctaccctt 720
tgacaatcag gtgctaatga ttgtatacta ttaaaaccag cacataagta ttgtaaatgt 780
gtgttcctcc taggttggaa gaaatgtctt tccttctatc tgggtcctgt taaagcgggt 840
gtcagttgtg tcttttcacc tcgatttgtg aattaataga attgggggga gaggaaatga 900
tgatgtcaat taagtttcag gtttggcatg atcatcattc tcgatgatat tctcactttg 960
tcgcaaatct gcccttatcg taagaacaag tttcagaatt ttccctccac tatacgactc 1020
cagtattatg tttacaatcc attggatgag tgcagcatta taagaccttg gtgcccagaa 1080

aaatctgtcc tttttggtac caaacctgag gtcttttgga agataatgta gaaaaccact 1140
acctattgaa ggcctgtttt ggctaatctg tgcaaactct gatgatacct gcttatgtgg 1200
attcttttcc acactgcttt catttttaag tataaagact tagaaaacta gaataatgct 1260
tttacaaata attaaaagta tgtgatgttc tgggtttttt ccttcttttt agaaccctgt 1320
atttaaacaa gccttctttt taagtcttgt ttgaaattta agtctcagat cttctggata 1380
ccaaatcaaa aacccaacgc gtaaaacagg gcagtatttg tgttcctaat tttaaaaagc 1440
tttatgtata ctctataaat atagatgcat aaacaacact tccccttgag tagcacatca 1500
acatacagca ttgtacatta caatgaaaat gtgtaactta agggtattat atatataaat 1560
acatatatac ctttgtaacc tttatactgt aaataaaaaa gttgctttag tcaaaaaaaa 1620

<210> 33
<211> 2968
<212> DNA
<213> Human

<400> 33

gaaaaagtag aaggaaacac agttcatata gaagtaaaag aaaaccctga agaggaggag 60
gaggaggaag aagaggaaga agaagatgaa gaaagtgaag aggaggagga agaggaggga 120
gaaagtgaag gcagtgaagg tgatgaggaa gatgaaaagg tgtcagatga gaaggattca 180
gggaagacat tagataaaaa gccaagtaaa gaaatgagct cagattctga atatgactct 240
gatgatgatc ggactaaaga agaaagggct tatgacaaag caaaacggag gattgagaaa 300
cggcgacttg aacatagtaa aaatgtaaac accgaaaagc taagagcccc tattatctgc 360
gtacttgggc atgtggacac agggaagaca aaaattctag ataagctccg tcacacacat 420
gtacaagatg gtgaagcagg tggtatcaca caacaaattg gggccaccaa tgttcctctt 480
gaagctatta atgaacagac taagatgatt aaaaattttg atagagagaa tgtacggatt 540
ccaggaatgc taattattga tactcctggg catgaatctt tcagtaatct gagaaataga 600
ggaagctctc tttgtgacat tgccatttta gttgttgata ttatgcatgg tttggagccc 660
cagacaattg agtctatcaa ccttctcaaa tctaaaaaat gtcccttcat tgttgcactc 720
aataagattg ataggttata tgattggaaa aagagtcctg actctgatgt ggctgctact 780
ttaaagaagc agaaaaagaa tacaaaagat gaatttgagg agcgagcaaa ggctattatt 840
gtagaatttg cacagcaggg tttgaatgct gctttgtttt atgagaataa agatccccgc 900
acttttgtgt ctttggtacc tacctctgca catactggtg atggcatggg aagtctgatc 960
taccttcttg tagagttaac tcagaccatg ttgagcaaga gacttgcaca ctgtgaagag 1020
ctgagagcac aggtgatgga ggttaaagct ctcccgggga tgggcaccac tatagatgtc 1080
atcttgatca atgggcgttt gaaggaagga gatacaatca ttgttcctgg agtagaaggg 1140
cccattgtaa ctcagattcg aggcctcctg ttacctcctc ctatgaagga attacgagtg 1200
aagaaccagt atgaaaagca taaagaagta gaagcagctc agggggtaaa gattcttgga 1260
aaagacctgg agaaaacatt ggctggttta cccctccttg tggcttataa agaagatgaa 1320
atccctgttc ttaaagatga attgatccat gagttaaagc agacactaaa tgctatcaaa 1380
ttagaagaaa aaggagtcta tgtccaggca tctacactgg gttctttgga agctctactg 1440
gaatttctga aaacatcaga agtgccctat gcaggaatta acattggccc agtgcataaa 1500
aaagatgtta tgaaggcttc agtgatgttg gaacatgacc ctcagtatgc agtaattttg 1560
gccttcgatg tgagaattga acgagatgca caagaaatgg ctgatagttt aggagttaga 1620
atttttagtg cagaaattat ttatcattta tttgatgcct ttacaaaata tagacaagac 1680
tacaagaaac agaaacaaga agaatttaag cacatagcag tatttccctg caagataaaa 1740
atcctccctc agtacatttt taattctcga gatccgatag tgatgggggt gacggtggaa 1800
gcaggtcagg tgaaacaggg gacacccatg tgtgtcccaa gcaaaaattt tgttgacatc 1860
ggaatagtaa caagtattga aataaaccat aaacaagtgg atgttgcaaa aaaaggacaa 1920
gaagtttgtg taaaaataga acctatccct ggtgagtcac ccaaaatgtt tggaagacat 1980
tttgaagcta cagatattct tgttagtaag atcagccggc agtccattga tgcactcaaa 2040
gactggttca gagatgaaat gcagaagagt gactggcagc ttattgtgga gctgaagaaa 2100
gtatttgaaa tcatctaatt ttttcacatg gagcaggaac tggagtaaat gcaatactgt 2160
gttgtaatat cccaacaaaa atcagacaaa aaatggaaca gacgtatttg gacactgatg 2220
gacttaagta tggaaggaag aaaaataggt gtataaaatg ttttccatga gaaaccaaga 2280
aacttacact ggtttgacag tggtcagtta catgtcccca cagttccaat gtgcctgttc 2340
actcacctct cccttcccca acccttctct acttggctgc tgttttaaag tttgcccttc 2400
cccaaatttg gatttttatt acagatctaa agctctttcg attttatact gattaaatca 2460
gtactgcagt atttgattaa aaaaaaaaaa gcagattttg tgattcttgg gacttttttg 2520
acgtaagaaa tacttcttta tttatgcata ttcttcccac agtgattttt ccagcattct 2580
tctgccatat gcctttaggg cttttataaa atagaaaatt aggcattctg atatttcttt 2640
agctgctttg tgtgaaacca tggtgtaaaa gcacagctgg ctgcttttta ctgcttgtgt 2700
agtcacgagt ccattgtaat catcacaatt ctaaaccaaa ctaccaataa agaaaacaga 2760
catccaccag taagcaagct ctgttaggct tccatggtta gtggtagctt ctctcccaca 2820
agttgtcctc ctaggacaag gaattatctt aacaaactaa actatccatc acactacctt 2880
ggtatgccag cacctgggta acagtaggag attttataca ttaatctgat ctgtttaatc 2940

tgatcggttt agtagagatt ttatacat

<210> 34
<211> 6011
<212> DNA
<213> Human

<400> 34

acggggcgcc ggacgacccg cacatcttat cctccacgcc ccactcgcac tcggagcggg 60
accgccccgg actccccctc gggccggcca ctcgaggagt gaggagagag gccgccggcc 120
cggcttgagc cgagcgcagc accccccgcg ccccgcgcca gaagtttggt tgaaccgggc 180
tgccgggaga aacttttttc ttttttcccc ctctcccggg agagtctctg gaggaggagg 240
ggaactcccc cggcccaagg ctcgtgggct cggggtcgcg cggccgcaga aggggcgggg 300
tccgcccgcg aggggaggcg cccccgggga cccgagaggg gggtgaggac cgcgggctgc 360
tggtgcggcg gcggcagcgt gtgccccgcg caggggaggc gccgccccgc tcccggcccg 420
gctgcgagga ggaggcggcg gcggcgcagg aggatgtact tggtggcggg ggacaggggg 480
ttggccggct gcgggcacct cctggtctcg ctgctggggc tgctgctgct gccggcgcgc 540
tccggcaccc gggcgctggt ctgcctgccc tgtgacgagt ccaagtgcga ggagcccagg 600
aaccgcccgg ggagcatcgt gcagggcgtc tgcggctgct gctacacgtg cgccagccag 660
gggaacgaga gctgcggcgg caccttcggg atttacggaa cctgcgaccg ggggctgcgt 720
tgtgtcatcc gccccccgct caatggcgac tccctcaccg agtacgaagc gggcgtttgc 780
gaagatgaga actggactga tgaccaactg cttggtttta aaccatgcaa tgaaaacctt 840
attgctggct gcaatataat caatgggaaa tgtgaatgta acaccattcg aacctgcagc 900
aatccctttg agtttccaag tcaggatatg tgcctttcag ctttaaagag aattgaagaa 960
gagaagccag attgctccaa ggcccgctgt gaagtccagt tctctccacg ttgtcctgaa 1020
gattctgttc tgatcgaggg ttatgctcct cctggggagt gctgtccctt acccagccgc 1080
tgcgtgtgca accccgcagg ctgtctgcgc aaagtctgcc agccgggaaa cctgaacata 1140
ctagtgtcaa aagcctcagg gaagccggga gagtgctgtg acctctatga gtgcaaacca 1200
gttttcggcg tggactgcag gactgtggaa tgccctactg ttcagcagac cgcgtgtccc 1260
ccggacagct atgaaactca agtcagacta actgcagatg gttgctgtac tttgccaaca 1320
agatgcgagt gtctctctgg cttatgtggt ttccccgtgt gtgaggtggg atccactccc 1380
cgcatagtct ctcgtggcga tgggacacct ggaaagtgct gtgatgtctt tgaatgtgtt 1440
aatgatacaa agccagcctg cgtatttaac aatgtggaat attatgatgg agacatgttt 1500
cgaatggaca actgtcggtt ctgtcgatgc caagggggcg ttgccatctg cttcaccgcc 1560
cagtgtggtg agataaactg cgagaggtac tacgtgcccg aaggagagtg ctgcccagtg 1620
tgtgaagatc cagtgtatcc ttttaataat cccgctggct gctatgccaa tggcctgatc 1680
cttgcccacg gagaccggtg gcgggaagac gactgcacat tctgccagtg cgtcaacggt 1740
gaacgccact gcgttgcgac cgtctgcgga cagacctgca caaaccctgt gaaagtgcct 1800
ggggagtgtt gccctgtgtg cgaagaacca accatcatca cagttgatcc acctgcatgt 1860
ggggagttat caaactgcac tctgacacgg aaggactgca ttaatggttt caaacgcgat 1920
cacaatggtt gtcggacctg tcagtgcata aacacccagg aactatgttc agaacgtaaa 1980
caaggctgca ccttgaactg tcccttcggt ttccttactg atgcccaaaa ctgtgagatc 2040
tgtgagtgcc gcccaaggcc caagaagtgc agacccataa tctgtgacaa gtattgtcca 2100
cttggattgc tgaagaataa gcacggctgt gacatctgtc gctgtaagaa atgtccagag 2160
ctctcatgca gtaagatctg ccccttgggt ttccagcagg acagtcacgg ctgtcttatc 2220
tgcaagtgca gagaggcctc tgcttcagct gggccaccca tcctgtcggg cacttgtctc 2280
accgtggatg gtcatcatca taaaaatgag gagagctggc acgatgggtg ccgggaatgc 2340
tactgtctca atggacggga aatgtgtgcc ctgatcacct gcccggtgcc tgcctgtggc 2400
aaccccacca ttcaccctgg acagtgctgc ccatcatgtg cagatgactt tgtggtgcag 2460
aagccagagc tcagtactcc ctccatttgc cacgcccctg gaggagaata ctttgtggaa 2520
ggagaaacgt ggaacattga ctcctgtact cagtgcacct gccacagcgg acgggtgctg 2580
tgtgagacag aggtgtgccc accgctgctc tgccagaacc cctcacgcac ccaggattcc 2640
tgctgcccac agtgtacaga tcaacctttt cggccttcct tgtcccgcaa taacagcgta 2700
cctaattact gcaaaaatga tgaaggggat atattcctgg cagctgagtc ctggaagcct 2760
gacgtttgta ccagctgcat ctgcattgat agcgtaatta gctgtttctc tgagtcctgc 2820
ccttctgtat cctgtgaaag acctgtcttg agaaaaggcc agtgttgtcc ctactgcata 2880
aaagacacaa ttccaaagaa ggtggtgtgc cacttcagtg ggaaggccta tgccgacgag 2940
gagcggtggg accttgacag ctgcacccac tgctactgcc tgcaggccca gaccctctgc 3000
tcgaccgtca gctgcccccc tctgccctgt gttgagccca tcaacgtgga aggaagttgc 3060
tgcccaatgt gtccagaaat gtatgtccca gaaccaacca atatacccat tgagaagaca 3120
aaccatcgag gagaggttga cctggaggtt cccctgtggc ccacgcctag tgaaaatgat 3180
atcgtccatc tccctagaga tatgggtcac ctccaggtag attacagaga taacaggctg 3240

cacccaagtg aagattcttc actggactcc attgcctcag ttgtggttcc cataattata 3300
tgcctctcta ttataatagc attcctattc atcaatcaga agaaacagtg gataccactg 3360
ctttgctggt atcgaacacc aactaagcct tcttccttaa ataatcagct agtatctgtg 3420
gactgcaaga aaggaaccag agtccaggtg gacagttccc agagaatgct aagaattgca 3480
gaaccagatg caagattcag tggcttctac agcatgcaaa aacagaacca tctacaggca 3540
gacaatttct accaaacagt gtgaagaaag gcaactagga tgaggtttca aaagacggaa 3600
gacgactaaa tctgctctaa aaagtaaact agaatttgtg cacttgctta gtggattgta 3660
ttggattgtg acttgatgta cagcgctaag accttactgg gatgggctct gtctacagca 3720
atgtgcagaa caagcattcc cacttttcct caagataact gaccaagtgt tttcttagaa 3780
ccaaagtttt taaagttgct aagatatatt tgcctgtaag atagctgtag agatatttgg 3840
ggtggggaca gtgagtttgg atggggaaag gggtgggagg gtggtgttgg gaagaaaaat 3900
tggtcagctt ggctcgggga gaaacctggt aacataaaag cagttcagtg gcccagaggt 3960
tatttttttc ctattgctct gaagactgca ctggttgctg caaagctcag gcctgaatga 4020
gcaggaaaca aaaaaggcct tgcgacccag ctgccataac caccttagaa ctaccagacg 4080
agcacatcag aaccctttga cagccatccc aggtctaaag ccacaagttt cttttctata 4140
cagtcacaac tgcagtaggc agtgaggaag ccagagaaat gcgatagcgg catttctcta 4200
aagcgggtta ttaaggatat atacagttac actttttgct gcttttattt tcttccaagc 4260
caatcaatca gccagttcct agcagagtca gcacatgaac aagatctaag tcatttcttg 4320
atgtgagcac tggagctttt tttttttaca acgtgacagg aagaggaggg agagggtgac 4380
gaacaccagg catttccagg ggctatattt cactgtttgt tgttgctttg ttctgttata 4440
ttgttggttg ttcatagttt ttgttgaagc tctagcttaa gaagaaactt tttttaaaaa 4500
gactgtttgg ggattctttt tccttattat atactgattc tacaaaatag aaactacttc 4560
attttaattg tatattattc aagcaccttt gttgaagctc aaaaaaaatg atgcctcttt 4620
aaactttagc aattatagga gtatttatgt aactatctta tgcttcaaaa aacaaaagta 4680
tttgtgtgca tgtgtatata atatatatat atacatatat atttatacac atacaattta 4740
tgttttcctg ttgaatgtat ttttatgaga ttttaaccag aacaaaggca gataaacagg 4800
cattccatag cagtgctttt gatcacttac aaattttttg aataacacaa aatctcattc 4860
tacctgcagt ttaattggaa agatgtgtgt gtgagagtat gtatgtgtgt gtgtgtgtgt 4920
gtgtgtgcgc gcgcacgcac gccttgagca gtcagcattg cacctgctat ggagaagggt 4980
attcctttat taaaatcttc ctcatttgga tttgctttca gttggttttc aatttgctca 5040
ctggccagag acattgatgg cagttcttat ctgcatcact aatcagctcc tggatttttt 5100
tttttttttt tcaaacaatg gtttgaaaca actactggaa tattgtccac aataagctgg 5160
aagtttgttg tagtatgcct caaatataac tgactgtata ctatagtggt aacttttcaa 5220
acagccctta gcacttttat actaattaac ccatttgtgc attgagtttt cttttaaaaa 5280
tgcttgttgt gaaagacaca gatacccagt atgcttaacg tgaaaagaaa atgtgttctg 5340
ttttgtaaag gaactttcaa gtattgttgt aaatacttgg acagaggttg ctgaacttta 5400
aaaaaaatta atttattatt ataatgacct aatttattaa tctgaagatt aaccattttt 5460
ttgtcttaga atatcaaaaa gaaaaagaaa aaggtgttct agctgtttgc atcaaaggaa 5520
aaaaagattt attatcaagg ggcaatattt ttatcttttc caaaataaat ttgttaatga 5580
tacattacaa aaatagattg acatcagcct gattagtata aattttgttg gtaattaatc 5640
cattcctggc ataaaaagtc tttatcaaaa aaaattgtag atgcttgctt tttgtttttt 5700
caatcatggc catattatga aaatactaac aggatatagg acaaggtgta aattttttta 5760
ttattatttt aaagatatga tttatcctga gtgctgtatc tattactctt ttactttggt 5820
tcctgttgtg ctcttgtaaa agaaaaatat aatttcctga agaataaaat agatatatgg 5880
cacttggagt gcatcatagt tctacagttt gtttttgttt tcttcaaaaa agctgtaaga 5940
gaattatctg caacttgatt cttggcagga aataaacatt ttgagttgaa atcaaaaaaa 6000
aaaaaaaaaa a

<210> 34a
<211> 1036
<212> DNA
<213> Human

<400> 34a

mylvagdrgl agcghllvsl lgllllpars gtralvclpc deskceeprn rpgsivqgvc 60
gccytcasqg nescggtfgi ygtcdrglrc virpplngds lteyeagvce denwtddqll 120
gfkpcnenli agcniingkc ecntirtcsn pfefpsqdmc lsalkrieee kpdcskarce 180
vqfsprcped svliegyapp geccplpsrc vcnpagclrk vcqpgnlnil vskasgkpge 240
ccdlyeckpv fgvdcrtvec ptvqqtacpp dsyetqvrlt adgcctlptr ceclsglcgf 300
pvcevgstpr ivsrgdgtpg kccdvfecvn dtkpacvfnn veyydgdmfr mdncrfcrcq 360
ggvaicftaq cgeinceryy vpegeccpvc edpvypfnnp agcyanglil ahgdrwredd 420
ctfcqcvnge rhcvatvcgq tctnpvkvpg eccpvceept iitvdppacg elsnctltrk 480
dcingfkrdh ngcrtcqcin tqelcserkq gctlncpfgf ltdaqnceic ecrprpkkcr 540

piicdkycpl gllknkhgcd icrckkcpel scskicplgf qqdshgclic kcreasasag 600
ppilsgtclt vdghhhknee swhdgcrecy clngremcal itcpvpacgn ptihpgqccp 660
scaddfvvqk pelstpsich apggeyfveg etwnidsctq ctchsgrvlc etevcppllc 720
qnpsrtqdsc cpqctdqpfr pslsrnnsvp nycknd egdi flaaeswkpd vctscicids 780
viscfsescp svscerpvlr kgqccpycik dtipkkvvch fsgkayadee rwdldscthc 840
yclqgqtlcs tvscpplpcv epinvegscc pmcpemyvpe ptnipiektn hrgevdlevp 900
lwptpsendi vhlprdmghl qvdyrdnrlh psedssldsi asvvvpiiic lsiiiaflfi 960
nqkkqwipll cwyrtptkps slnnqlvsvd ckkgtrvqvd ssqrmlriae pdarfsgfys 1020
mqkqnhlqad nfyqtv

<210> 35
<211> 716
<212> DNA
<213> Human

<400> 35

gcagtacctg gagtgtcctg cagggggaaa gcgaaccggg ccctgaagtc cggggcagtc 60
acccggggct cctgggccgc tctgccgggc tggggctgag cagcgatcct gctttgtccc 120
agaagtccag agggatcagc cccagaacac accctcctcc ccgggacgcc gcagctttct 180
ggaggctgag gaaggcatga agagtgggct ccacctgctg gccgactgag aaaagaattt 240
ccagaactcg gtcctatttt acagattgag aaactatggt tcaagaagag aggacggggc 300
ttgagggaat ctcctgattc tccttatatg acctcaaact gaccatacta aacagtgtag 360
aaggtctttt taaggctcta aatgtcaggg tctcccatcc cctgatgcct gacttgtaca 420
gtcagtgtgg agtagacggt ttcctccacc cagggttgac tcagggggat gatctgggtc 480
ccattctggt cttaagaccc caaacaaggg ttttttcagc tccaggatct ggagcctcta 540
tctggttagt gtcgtaacct ctgtgtgcct cccgttaccc catctgtcca gtgagctcag 600
cccccatcca cctaacaggg tggccacagg gattactgag ggttaagacc ttagaactgg 660
gtctagcacc cgataagagc tcaataaatg ttgttccttt ccacatcaaa aaaaaa

<210> 36
<211> 395
<212> DNA
<213> Human

<400> 36

ccaatacttc attcttcatt ggtggagaag attgtagact tctaagcatt ttccaaataa 60
aaaagctatg atttgatttc caacttttaa acattgcatg tcctttgcca tttactacat 120
tctccaaaaa aaccttgaaa tgaagaaggc cacccttaaa atacttcaga ggctgaaaat 180
atgattatta cattggaatc ctttagccta tgtgatattt ctttaacttt gcactttcac 240
gcccagtaaa accaaagtca gggtaaccaa tgtcatttta caaaatgtta aaaccctaat 300
tgcagttcct tttttaaatt attttaaaga ttacttaaca acattagaca gtgcaaaaaa 360
agaagcaagg aaagcattct taattctacc atcct

<210> 37
<211> 134
<212> DNA
<213> Human

<400> 37

ccctcgagcg gccgcccggg caggtacttt taccaccgaa ttgttcactt gactttaaga 60
aacccataaa gctgcctggc tttcagcaac aggcctatca acaccatggt gagtctccat 120
aagggacacc gtgt

<210> 38
<211> 644
<212> DNA
<213> Human

<400> 38

aagcctgttg tcatggggga ggtggtggcg cttggtggcc actggcggcc gaggtagagg 60

cagtggcgct tgagttggtc gggggcagcg gcagatttga ggcttaagca acttcttccg 120
gggaagagtg ccagtgcagc cactgttaca attcaagatc ttgatctata tccatagatt 180
ggaatattgg tgggccagca atcctcagac gcctcactta ggacaaatga ggaaactgag 240
gcttggtgaa gttacgaaac ttgtccaaaa tcacacaact tgtaaagggc acagccaaga 300
ttcagagcca ggctgtaaaa attaaaatga acaaattacg gcaaagtttt aggagaaaga 360
aggatgttta tgttccagag gccagtcgtc cacatcagtg gcagacagat gaagaaggcg 420
ttcgcaccgg aaaatgtagc ttcccggtta agtaccttgg ccatgtagaa gttgatgaat 480
caagaggaat gcacatctgt gaagatgctg taaaaagatt gaaagctgaa aggaagttct 540
tcaaaggctt ctttggaaaa actggaaaga aagcagttaa agcagtttct gtgggtctaa 600
gcagatggac tcagaggttg tggatgaaaa actaaggacc tcat

<210> 39
<211> 657
<212> DNA
<213> Human

<400> 39

cttttgtttt gggttttcca atgtagatgt ctcagtgaaa tgtgcagata tactttgttc 60
cttatatggt caccagtgtt aattatggac aaatacatta aaacaagggt tcctggccca 120
gcctcccatc taatctcttt gatactcttg gaatctaagt ctgaggagcg atttctgaat 180
tagccagtgt tgtaccaact ttctgttagg aattgtatta gaataacctt tctttttcag 240
acctgctcag tgagacatct tggggaatga agtaggaaaa tagacatttg gtggaaaaac 300
agcaaaatga gaacattaaa aagactcatt caagtatgag tataaagggc atggaaattc 360
tggtcctttg agcaaaatga gaagaaaaaa ttctgctcag cagtattcac tgtgttaaga 420
tttttgtttt tttacacgaa tggaaaaatg atgtgtaagt ggtatagatt ttaatcagct 480
aacagtcact ccagagattt tgatcagcac caattcctat agtagtaagt atttaaaagt 540
taagaaatac tactacattt aacattataa agtagagttc tggacataac tgaaaattag 600
atgtttgctt caatagaaat ttgttcccac ttgtattttc aacaaaatta tcggaac

<210> 40
<211> 1328
<212> DNA
<213> Human

<400> 40

acaattttaa aataactagc aattaatcac agcatatcag gaaaaagtac acagtgagtt 60
ctggttagtt tttgtaggct cattatggtt agggtcgtta agatgtatat aagaacctac 120
ctatcatgct gtatgtatca ctcattccat tttcatgttc catgcatact cgggcatcat 180
gctaatatgt atccttttaa gcactctcaa ggaaacaaaa gggcctttta tttttataaa 240
ggtaaaaaaa attccccaaa tattttgcac tgaatgtacc aaaggtgaag ggacattaca 300
atatgactaa cagcaactcc atcacttgag aagtataata gaaaatagct tctaaatcaa 360
acttccttca cagtgccgtg tctaccacta caaggactgt gcatctaagt aataattttt 420
taagattcac tatatgtgat agtatgatat gcatttattt aaaatgcatt agactctctt 480
ccatccatca aatactttac aggatggcat ttaatacaga tatttcgtat ttcccccact 540
gctttttatt tgtacagcat cattaaacac taagctcagt taaggagcca tcagcaacac 600
tgaagagatc agtagtaaga attccatttt ccctcatcag tgaagacacc acaaattgaa 660
actcagaact atatttctaa gcctgcattt tcactgatgc ataattttct tagtaatatt 720
aagagacagt ttttctatgg catctccaaa actgcatgac atcactagtc ttacttctgc 780
ttaattttat gagaaggtat tcttcatttt aattgctttt gggattactc cacatctttg 840
tttatttctt gactaatcag attttcaata gagtgaagtt aaattggggg tcataaaagc 900
attggattga catatggttt gccagcctat gggtttacag gcattgccca aacatttctt 960
tgagatctat atttataagc agccatggaa ttcctattat gggatgttgg caatcttaca 1020
ttttatagag gtcatatgca tagttttcat aggtgttttg taagaactga ttgctctcct 1080
gtgagttaag ctatgtttac tactgggacc ctcaagagga ataccactta tgttacactc 1140
ctgcactaaa ggcacgtact gcagtgtgaa gaaatgttct gaaaaagggt tatagaaatc 1200
tggaaataag aaaggaagag ctctctgtat tctataattg gaagagaaaa aaagaaaaac 1260
ttttaactgg aaatgttagt ttgtacttat tgatcatgaa tacaagtata tatttaattt 1320
tgaaaaaa

<210> 41
<211> 987
<212> DNA
<213> Human

<400> 41
   aacagagact ggcacaggac ctcttcattg caggaagatg gtagtgtagg caggtaacat 60
   tgagctcttt tcaaaaaagg agagctcttc ttcaagataa ggaagtggta gttatggtgg 120
   taacccccgg ctatcagtcc ggatggttgc cacccctcct gctgtaggat ggaagcagcc 180
   atggagtggg agggaggcgc aataagacac ccctccacag agcttggcat catgggaagc 240
   tggttctacc tcttcctggc tcctttgttt aaaggcctgg ctgggagcct tccttttggg 300
   tgtctttctc ttctccaacc aacagaaaag actgctcttc aaaggtggag ggtcttcatg 360
   aaacacagct gccaggagcc caggcacagg gctgggggcc tggaaaaagg agggcacaca 420
   ggaggaggga ggagctggta gggagatgct ggctttacct aaggtctcga aacaaggagg 480
   gcagaatagg cagaggcctc tccgtcccag gcccattttt gacagatggc gggacggaaa 540
   tgcaatagac cagcctgcaa gaaagacatg tgttttgatg acaggcagtg tggccgggtg 600
   gaacaagcac aggccttgga atccaatgga ctgaatcaga accctaggcc tgccatctgt 660
   cagccgggtg acctgggtca attttagcct ctaaaagcct cagtctcctt atctgcaaaa 720
   tgaggcttgt gatacctgtt ttgaagggtt gctgagaaaa ttaaagataa gggtatccaa 780
   aatagtctac ggccatacca ccctgaacgt gcctaatctc gtaagctaag caggtcaggg 840
   cctggttagt acctggatgg ggagagtatg gaaaacatac ctgcccgcag ttggagttgg 900
   actctgtctt aacagtagcg tggcacacag aaggcactca gtaaatactt gttgaataaa 960
   tgaagtagcg atttggtgtg aaaaaaa

<210> 42
<211> 956
<212> DNA
<213> Human

<400> 42
   cggacggtgg ggcggacgcg tgggtgcagg agcagggcgg ctgccgactg ccccaaccaa 60
   ggaaggagcc cctgagtccg cctgcgcctc catccatctg tccggccaga gccggcatcc 120
   ttgcctgtct aaagccttaa ctaagactcc cgccccgggc tggccctgtg cagaccttac 180
   tcaggggatg tttacctggt gctcgggaag ggaggggaag ggccggggga gggggcacgg 240
   caggcgtgtg gcagccacac gcaggcggcc agggcggcca gggacccaaa gcaggatgac 300
   cacgcacctc cacgccactg cctcccccga atgcatttgg aaccaaagtc taaactgagc 360
   tcgcagcccc cgcgccctcc ctccgcctcc catcccgctt agcgctctgg acagatggac 420
   gcaggccctg tccagccccc agtgcgctcg ttccggtccc cacagactgc cccagccaac 480
   gagattgctg gaaaccaagt caggccaggt gggcggacaa aagggccagg tgcggcctgg 540
   ggggaacgga tgctccgagg actggactgt ttttttcaca catcgttgcc gcagcggtgg 600
   gaaggaaagg cagatgtaaa tgatgtgttg gtttacaggg tatatttttg ataccttcaa 660
   tgaattaatt cagatgtttt acgcaaggaa ggacttaccc agtattactg ctgctgtgct 720
   tttgatctct gcttaccgtt caagaggcgt gtgcaggccg acagtcggtg accccatcac 780
   tcgcaggacc aagggggcgg ggactgctgg ctcacgcccc gctgtgtcct ccctcccctc 840
   ccttccttgg gcagaatgaa ttcgatgcgt attctgtggc cgccatctgc gcagggtggt 900
   ggtattctgt catttacaca cgtcgttcta attaaaaagc gaattatact ccaaaa

<210> 43
<211> 536
<212> DNA
<213> Human

<400> 43

aaataaacac ttccataaca ttttgttttc gaagtctatt aatgcaatcc cacttttttc 60
cccctagttt ctaaatgtta aagagagggg aaaaaaggct caggatagtt ttcacctcac 120
agtgttagct gtcttttatt ttactcttgg aaatagagac tccattaggg ttttgacatt 180
ttgggaaccc agttttacca ttgtgtcagt aaaacaataa gatagtttga gagcatatga 240
tctaaataaa gacatttgaa gggttagttt gaattctaaa agtaggtaat agccaaatag 300
cattctcatc ccttaacaga caaaaactta tttgtcaaaa gaattagaaa aggtgaaaat 360
attttttcca gatgaaactt gtgccacttc caattgacta atgaaataca aggagacaga 420
ctggaaaaag tgggttatgc cacctttaaa accctttctg gtaaatatta tggtagctaa 480
agggtggttt ccccggcacc tggacctgga caggtagggt tccgtggtta accagt

<210> 44
<211> 1630
<212> DNA
<213> Human

<400> 44

ggggagggac gagtatggaa ccctgaaggt agcaagtcca ggcactggcc tgaccatccg 60
gctccctggg caccaagtcc caggcaggag cagctgtttt ccatcccttc ccagacaagc 120
tctattttta tcacaatgac ctttagagag gtctcccagg ccagctcaag gtgtcccact 180
atcccctctg gagggaagag gcaggaaaat tctccccggg tccctgtcat gctactttct 240
ccatcccagt tcagactgtc caggacatct tatctgcagc cataagagaa ttataaggca 300
gtgatttccc ttaggcccag gacttgggcc tccagctcat ctgttccttc tgggcccatt 360
catggcaggt tctgggctca aagctgaact ggggagagaa gagatacaga gctaccatgt 420
gactttacct gattgccctc agtttggggt tgcttattgg gaaagagaga gacaaagagt 480
tacttgttac gggaaatatg aaaagcatgg ccaggatgca tagaggagat tctagcaggg 540
gacaggattg gctcagatga cccctgaggg ctcttccagt cttgaaatgc attccatgat 600
attaggaagt cgggggtggg tggtggtggt gggctagttg ggtttgaatt taggggccga 660
tgagcttggg tacgtgagca gggtgttaag ttagggtctg cctgtatttc tggtcccctt 720
ggaaatgtcc ccttcttcag tgtcagacct cagtcccagt gtccatatcg tgcccagaaa 780
agtagacatt atcctgcccc atcccttccc cagtgcactc tgacctagct agtgcctggt 840
gcccagtgac ctgggggagc ctggctgcag gccctcactg gttccctaaa ccttggtggc 900
tgtgattcag gtccccaggg gggactcagg gaggaatatg gctgagttct gtagtttcca 960
gagttggctg gtagagcctt ctagaggttc agaatattag cttcaggatc agctgggggt 1020
atggaattgg ctgaggatca aacgtatgta ggtgaaagga taccaggatg ttgctaaagg 1080
tgagggacag tttgggtttg ggacttacca gggtgatgtt agatctggaa cccccaagtg 1140
aggctggagg gagttaaggt cagtatggaa gatagggttg ggacagggtg ctttggaatg 1200
aaagagtgac cttagagggc tccttgggcc tcaggaatgc tcctgctgct gtgaagatga 1260
gaaggtgctc ttactcagtt aatgatgagt gactatattt accaaagccc ctacctgctg 1320
ctgggtccct tgtagcacag gagactgggg ctaagggccc ctcccaggga agggacacca 1380
tcaggcctct ggctgaggca gtagcataga ggatccattt ctacctgcat ttcccagagg 1440
actagcagga ggcagccttg agaaaccggc agttcccaag ccagcgcctg gctgttctct 1500
cattgtcact gccctctccc caacctctcc tctaacccac tagagattgc ctgtgtcctg 1560
cctcttgcct cttgtagaat gcagctctgg ccctcaataa atgcttcctg cattcatctg 1620
caaaaaaaaa

<210> 45
<211> 169
<212> DNA
<213> Human

<400> 45

tcttttgctt ttagcttttt atttttgtat taacaggagt cttattacac ataggtctga 60
taaaactggt ttatgatctt cagtctgatt ccagtgctgc ataactagat aacgtatgaa 120
ggaaaaacga cgacgaacaa aaaagtaagt gcttggaaga cttagttga

<210> 46
<211> 769
<212> DNA
<213> Human

<400> 46

tgcaggtcat atttactatc ggcaataaaa ggaagcaaag cagtattaag cagcggtgga 60
atttgtcgct ttcacttttt ataaagtgct acataaaatg tcatatttcc aaatttaaaa 120
acataactcc agttcttacc atgagaacag catggtgatc acgaaggatc ttcttgaaaa 180
aaacaaaaac aaaaacaaaa aacaatgatc tcttctgggt atcacatcaa atgagataca 240
aaggtgtact aggcaatctt agagatctgg caacttattt tatatataag gcatctgtga 300
ccaagagacg ttatgaatta aatgtacaaa tgtattatgt ataaatgtat taaatgcaag 360
cttcatataa tgacaccaat gtctctaagt tgctcagaga tcttgactgg ctgtggccct 420
ggccagctcc tttcctgata gtctgattct gccttcatat ataggcagct cctgatcatc 480
catgccagtg aatgagaaaa caagcatgga atatataaac tttaacatta aaaaatgttt 540
tattttgtaa taaaatcaaa tttcccattg aaaccttcaa aaactttgca gaatgaggtt 600
ttgatatatg tgtacaagta gtaccttctt agtgcaagaa aacatcatta tttctgtctg 660
cctgcctttt tgtttttaaa aatgaagact atcattgaaa caagtttgtc ttcagtatca 720
ggacatgttg acggagagga aaggtaggaa agggttaggg atagaagcc

<210> 47
<211> 2529
<212> DNA

<213> Human

<400> 47

tttagttcat agtaatgtaa aaccatttgt ttaattctaa atcaaatcac tttcacaaca 60
gtgaaaatta gtgactggtt aaggtgtgcc actgtacata tcatcatttt ctgactgggg 120
tcaggacctg gtcctagtcc acaaggtggg caggaggagg gtggaggcta agaacacaga 180
aaacacacaa aagaaaggaa agctgccttg gcagaaggat gaggtggtga gcttgccgag 240
ggatggtggg aagggggctc cctgttgggg ccgagccagg agtcccaagt cagctctcct 300
gccttactta gctcctggca gagggtgagt ggggacctac gaggttcaaa atcaaatggc 360
atttggccag cctggcttta ctaacaggtt cccagagtgc ctctgttggc tgagctctcc 420
tgggctcact ccatttcatt gaagagtcca aatgattcat tttcctaccc acaacttttc 480
attattcttc tggaaaccca tttctgttga gtccatctga cttaagtcct ctctccctcc 540
actagttggg gccactgcac tgaggggggt cccaccaatt ctctctagag aagagacact 600
ccagaggccc ctgcaacttt gcggatttcc agaaggtgat aaaaagagca ctcttgagtg 660
ggtgcccagg aatgtttaaa atctatcagg cacactataa agctggtggt ttcttcctac 720
caagtggatt cggcatatga accacctact caatacttta tattttgtct gtttaaacac 780
tgaactctgg tgttgacagg tacaaaggag aagagatggg gactgtgaag aggggagggc 840
ttccctcatc ttcctcaaga tctttgtttc cataaactat gcagtcataa ttgagaaaaa 900
gcaatagatg gggcttccta ccatttgttg gttattgctg gggttagcca ggagcagtgt 960
ggatggcaaa gtaggagaga ggcccagagg aaagcccatc tccctccagc tttggggtct 1020
ccagaaagag gctggatttc tgggatgaag cctagaaggc agagcaagaa ctgttccacc 1080
aggtgaacag tcctacctgc ttggtaccat agtccctcaa taagattcag aggaagaagc 1140
ttatgaaact gaaaatcaaa tcaaggtatt gggaagaata atttcccctc gattccacag 1200
gagggaagac cacacaatat cattgtgctg gggctcccca aggccctgcc acctggcttt 1260
acaaatcatc aggggttgcc tgcttggcag tcacatgctt ccctggtttt agcacacata 1320
caaggagttt tcagggaact ctatcaagcc ataccaaaat cagggtcaca tgtgggtttc 1380
ccctttcctt gcctcttcat aaaagacaac ttggcttctg aggatggtgg tcttttgcat 1440
gcagttgggc tgacctgaca aagcccccag tttcctgtgg caggttctgg gagaggatgc 1500
attcaagctt ctgcagccta ggggacaggg ctgcttgttc agttattact gcctcggagc 1560
tccaaatccc accaaagtcc tgactccagg tctttcctaa tgcacagtag tcagtctcag 1620
cttcggcagt attctcggct gtatgttctc tggcagagag aggcagatga acatagtttt 1680
agggagaaag ctgatgggaa acctgtgagt taagccacat gtctcaccag gaataattta 1740
tgccaggaaa ccaggaagtc attcaagttg ttctctgagg ccaaagacac tgagcacagc 1800
ccagagccaa taaaagatct ttgagtctct ggtgaattca cgaagtgacc ccagctttag 1860
ctactgcaat tatgattttt atgggacagc aatttcttgc atctctacag aggaagaaga 1920
gggggagtgg gaggggaagg aaagagaaca gagcggcact gggatttgaa aggggaacct 1980
ctctatctga ggagccccca ctggcttcag aagcaactta ccaaggggta tttaaagaca 2040
tgaaaatttc cagaaatacc atttggtgca tccctttgtt tctgtaatat taaactcagg 2100
tgaaattata ctctgacagt ttctctcttt ctgcctcttc cctctgcaga gtcaggacct 2160
gcagaactgg ctgaaacaag atttcatggt gtcacccatg agagatgact caatgccaag 2220
gcctgaagtt atagagtgtt tacagcggtg gcgatattca ggggtcatcg ccaactggtc 2280
tcgagttcca aagctctgat gaagaaacaa gactccttga tgtgttactg atcccactga 2340
ttccaggagt caagattagc caggaagcca aacaccagga gttggggtgg cacgtcacca 2400
gtccagagcc ctgccacgga tgtacgcagg agcccagcat taggcaatca ggagccagaa 2460
catgatcacc agggccacaa ataggaagag gcgtgacagg aactgctcgt ccacatacct 2520
ggggtgtcc

<210> 48
<211> 1553
<212> DNA
<213> Human

<400> 48

tttttttttt tttttgattt ctgggacaat taagctttat ttttcatata tatatatatt 60
ttcatatata tatatacata catatataaa ggaaacaatt tgcaaattta cacacctgac 120
aaaaccatat atacacacat atgtatgcat acacacagac agacacacac acccgaagct 180
ctagccaggc ccgttttcca tccctaagta ccattctctc atttgggccc ttctagggtt 240
ggggccctga gcttggtttg tagaagtttg gtgctaatat aaccatagct ttaatcccca 300
tgaaggacag tgtagacctc atctttgtct gctccccgct gcctttcagt tttacgtgat 360
ccatcaagag ggctatggga gccaagtgaa cacgggggat tgaggctaat tcacctgaac 420
tcgaaaacag cgcccagctt cctcaccgca ggcacgcgtc ttttcttttt ttttcctcga 480
gacggagtct cgctgtgttg cccaggctgg agtgcagtgg cacggtctcg gctcactgca 540
agctccacct cctggattca taccattctc ctgcttcagc cttccgagta gctgggacta 600
taggtgccaa ccactacgcc tagctaattt ttttttgtat ttttagtaga gacagggttt 660

cacc gtgtta gccaggatgg tctcgtcctg actttgtgat ccgcccgcct cggcctccca 720
aagtgctggg attacaggcg tgagccacca cacctggccc cggcacgtat cttttaagga 780
atgacaccag ttcctggctt ctgaccaaag aaaaaatgtc acaggagact ttgaagaggc 840
agacaggagg gtggtggcag caacactgca gctgcttctg gatgctgctg gggtgctctc 900
cggagcgggt gtgaacagcg cacttcaaca tgagcaggcg cctggctccg gtgtgtcctc 960
acttcagtgg tgcacctgga tggtggaagc cagcctttgg ggcaggaaac cagctcagag 1020
aggctaccca gctcagctgc tggcaggagc caggtattta cagccataat gtgtgtaaag 1080
aaaaaacacg ttctgcaaga aactctccta cccgctcggg agactggggc tccttgcttg 1140
ggatgagctt cactcaacgt ggagatggtg gtggactggt ccctgaaaag cgggccttgc 1200
agggccaagt gaggtcctca ggtcctaac ccagtggccc tctgaaaggg ggtgtgcagg 1260
cgaggggagc aggaggcttc tctctagtcc ctttggaggc tttggctgag agaagagtga 1320
gcagggagct gggaatggtc caggcaggga agggagctga agtgattcgg ggctaatgcc 1380
tcagatcgat gtatttctct ccctggtctc ccggagccct cttgtcaccg ctgctgccct 1440
gcaggaggcc catctcttct gggagcttat ctgacttaac ttcaactaca agttcgctct 1500
tacgagaccg ggggtagcgt gatctcctgc ttccctgagc gcctgcacgg cag

<210> 49
<211> 921
<212> DNA
<213> Human

<400> 49

ctgtggtccc agctactcag gaggctgagg cgggaggatt gcttgagccc aggagttgga 60
tgttgcagtg agccaagatc gcaccattgc cctccactct gggccacgga gcaataccct 120
gtctcagaaa acaaacaaca aaaagcagaa acgctgaagg ggtcggttta cgggaaaacc 180
gcctgtcaga acacttggct actcctaccc cagatcagtg gacctgggaa tgagggttgg 240
tcccgggagg cttttctcca agctgttgcc accagacccg ccatgggaac cctggccaca 300
gaagcctccc ggggagtgag ccagagcctg gaccgctgtg ctgatgtgtc tggggtggag 360
ggagggtggg gagtgtgcaa gggtgtgtgt gtgcccgggg ggtgttcatg ggcaagcatg 420
tgcgtgcctg tgtgtgtgcg tgcccctccc ctgcagccgt cggtggtatc tccctccagc 480
cccttcgcca ccttctgagc attgtctgtc cacgtgagac tgcccagaga cagcagagct 540
ccacgtggtt ttaaggggag acctttccct ggacctgggg gtctcgccgt atctcatgac 600
caggtgctaa atgacccgac atgcatcacc tgcctttcga tgaccaacct ccctgtcccc 660
gtcccgctga cctgcccccg tggcgtctca cggtgatgcc tgctcctgac attggtgttc 720
actgtagcaa actacattct ggatgggaat tttcatgtac atgtgtggca tgtggaaaat 780
ttcaaataaa atggacttga tttagaaagc caaaaagctg tgtggtcctt ccagcacgga 840
tactttgacc tcttgcctac aaccccttcc ttgggtccga ggctggtagc tttgttcact 900
tcagatggtt gggggcgggt g

<210> 50
<211> 338
<212> DNA
<213> Human

<400> 50

atgatctatc tagatgccct accgtaaaat caaaacacaa aaccctactg actcattccc 60
tcccttccag atattacccc atttctctac ttcccattgt agccaaactt tccaaaaatt 120
catgttctgt cttcatttcc tcatgttcaa cccaccctgt cttagctacc acccctcagt 180
aacgacctag cctgggtaga aacaaatgtc agcatgatac catactcaat gatccttcgt 240
cactgttgtc attgtcatca ttccatggcc ttactttccc tctcagcgcc atttgctaca 300
gtaagaaact ttctttcttg aattcttggt tctcttgg

<210> 51
<211> 1191
<212> DNA
<213> Human

<400> 51

ctagcaagca ggtaaacgag ctttgtacaa acacacacag accaacacat ccggggatgg 60
ctgtgtgttg ctagagcaga ggctgattaa acactcagtg tgttggctct ctgtgccact 120
cctggaaaat aatgaattgg gtaaggaaca gttaataaga aaatgtgcct tgctaactgt 180
gcacattaca acaaagagct ggcagctcct gaaggaaaag ggcttgtgcc gctgccgttc 240
aaacttgtca gtcaactcat gccagcagcc tcagcgtctg cctccccagc acaccctcat 300

tacatgtgtc tgtctggcct gatctgtgca tctgctcgga gacgctcctg acaagtcggg 360
aatttctcta tttctccact ggtgcaaaga gcggatttct ccctgcttct cttctgtcac 420
ccccgctcct ctcccccagg aggctccttg atttatggta gctttggact tgcttccccg 480
tctgactgtc cttgacttct agaatggaag aagctgagct ggtgaaggga agactccagg 540
ccatcacaga taaaagaaaa atacaggaag aaatctcaca gaagcgtctg aaaatagagg 600
aagacaaact aaagcaccag catttgaaga aaaaggcctt gagggagaaa tggcttctag 660
atggaatcag cagcggaaaa gaacaggaag agatgaagaa gcaaaatcaa caagaccagc 720
accagatcca ggttctagaa caaagtatcc tcaggcttga gaaagagatc caagatcttg 780
aaaaagctga actgcaaatc tcaacgaagg aagaggccat tttaaagaaa ctaaagtcaa 840
ttgagcggac aacagaagac attataagat ctgtgaaagt ggaaagagaa gaaagagcag 900
aagagtcaat tgaggacatc tatgctaata tccctgacct tccaaagtcc tacatacctt 960
ctaggttaag gaaggagata aatgaagaaa aagaagatga tgaacaaaat aggaaagctt 1020
tatatgccat ggaaattaaa gttgaaaaag acttgaagac tggagaaagt acagttctgt 1080
cttccaatac ctctggccat cagatgactt taaaaggtac aggagtaaaa gtttaagatg 1140
atgggcaaaa gtccagtgta ttcagtaaag tgctaatcac aagttggagg t

<210> 52
<211> 1200
<212> DNA
<213> Human

<400> 52

aacagggact ctcactctat caaccccagg ctggagtccg gtgcgcccac cctggctccc 60
tgcaacctcc gcctcccagg ctcaagcaac tctcctgcct cagtcgctct agtagctggg 120
actacaggca cacaccacca tgcccagcca atttttgcat tttttgtaga gacagggttt 180
cgccttctgt ccaggccggc atcatatact ttaaatcatg cccagatgac tttaatacct 240
aatacaatat atcaggttgg tttaaaaata attgcttttt tattattttt gcatttttgc 300
accaacctta atgctatgta aatagttgtt atactgttgc ttaacaacag tatgacaatt 360
ttggcttttt ctttgtatta ttttgtattt ttttttttta ttgtgtggtc tttttttttt 420
ttctcagtgt tttcaattcc tccttggttg aatccatgga tgcaaaaccc acagatatga 480
agggctggct atatatgcat tgatgattgt cctattatat tagttataaa gtgtcattta 540
atatgtagtg aaagttatgg tacagtggaa agagtagttg aaaacataaa catttggacc 600
tttcaagaaa ggtagcttgg tgaagttttt caccttcaaa ctatgtccca gtcagggctc 660
tgctactaat tagctataat ctttgcacaa attacatcac ctttgagtct cagttgcctc 720
acctgtaaaa tgaaagaact ggatactctc taaggtcact tccagccctg tcattctata 780
actctgttat gctgaggaag aaattcacat tgtgttaact gtatgagtca aactgaaaat 840
gattattaaa gtgggaaaaa gccaattgct tctcttagaa agctcaacta aatttgagaa 900
gaataatctt ttcaattttt taagaattta aatattttta agggtttgac ctatttattt 960
agagatgggg tctcactctg tcacccagac tggagtacag tggcacaatc atagctcact 1020
gctgcctcaa attcatgggc tcaagtgatc ctcctgcctc tgcctccaga gtagctgcga 1080
ctatgggcat gtgccaccac gcctggctaa catttgtatt gacctattta tttattgtga 1140
tttatatctt tttttttttt tctttttttt ttttttacaa aatcagaaat acttattttg 1200

<210> 53
<211> 989
<212> DNA
<213> Human

<400> 53

aagccaccac tcaaaacttc ctatacattt tcacagcaga gacaagtgaa catttatttt 60
tatgcctttc ttcctatgtg tatttcaagt ctttttcaaa acaaggcccc aggactctcc 120
gattcaatta gtccttgggc tggtcgactg tgcaggagtc cagggagcct ctacaaatgc 180
agagtgactc tttaccaaca taaaccctag atacatgcaa aaagcaggac ccttcctcca 240
ggaatgtgcc atttcagatg cacagcaccc atgcagaaaa gctggaattt tccttggaac 300
cgactgtgat agaggtgctt acatgaacat tgctactgtc tttctttttt tttgagacag 360
gtttcgcttg tgcccaggct gagtgcaatg cgtgatctca ctcactgcaa ttccacctcc 420
aggttcaagc attctcctgc tcagcctcct agtagctggg ttacaggcac tgccaccatg 480
ccggctaatt ttgtattttt gtagagatgg atttctccat ttggtcaggc ggtctcgaac 540
cccaacctca gtgatctgcc acctcagcct cctaagtgtt ggattacagg atgagccacc 600
cgaccggcca ctactgtctt tctttgaccc ttccagtttc gaagataaag aggaaataat 660
ttctctgaag tacttgataa aatttccaaa caaaacacat gtccacttca ctgataaaaa 720
atttaccgca gtttggcacc taagagtatg acaacagcaa taaaaagtaa tttcaaagag 780
ttaagatttc ttcagcaaaa tagatgattc acatcttcaa gtcctttttg aaatcagtta 840
ttaatattat tctttcctca tttccatctg aatgactgca gcaatagttt tttttttttt 900

tttttttttt ttgcgagatg gaatctcgct ctgtcgccca gcgggagtgc actggcgcaa 960
gcccggctca ccgcaatctc tgccacccg

<210> 54
<211> 250
<212> DNA
<213> Human

<400> 54

catttcccca ttggtcctga tgttgaagat ttagttaaag aggctgtaag tcaggttcga 60
gcagaggcta ctacaagaag tagggaatca agtccctcac atgggctatt aaaactaggt 120
agtggtggag tagtgaaaaa gaaatctgag caacttcata acgtaactgc ctttcaggga 180
aaagggcatt ctttaggaac tgcatctggt aacccacacc ttgatccaag agctagggaa 240
acttcagttg

<210> 55
<211> 2270
<212> DNA
<213> Human

<400> 55

gcgcccccga gcagcgcccg cgccctccgc gccttctccg ccgggacctc gagcgaaaga 60
ggcccgcgcg ccgcccagcc ctcgcctccc tgcccaccgg gcacaccgcg ccgccacccc 120
gaccccgctg cgcacggcct gtccgctgca caccagcttg ttggcgtctt cgtcgccgcg 180
ctcgccccgg gctactcctg cgcgccacaa tgagctcccg catcgccagg gcgctcgcct 240
tagtcgtcac ccttctccac ttgaccaggc tggcgctctc cacctgcccc gctgcctgcc 300
actgccccct ggaggcgccc aagtgcgcgc cgggagtcgg gctggtccgg gacggctgcg 360
gctgctgtaa ggtctgcgcc aagcagctca acgaggactg cagcaaaacg cagccctgcg 420
accacaccaa ggggctggaa tgcaacttcg gcgccaagtc caccgctctg aaggggatct 480
gcagagctca gtcagagggc agaccctgtg aatataactc cagaatctac caaaacgggg 540
aaagtttcca gcccaactgt aaacatcagt gcacatgtat tgatggcgcc gtgggctgca 600
ttcctctgtg tccccaagaa ctatctctcc ccaacttggg ctgtcccaac cctcggctgg 660
tcaaagttac cgggcagtgc tgcgaggagt gggtctgtga cgaggatagt atcaaggacc 720
ccatggagga ccaggacggc ctccttggca aggagctggg attcgatgcc tccgaggtgg 780
agttgacgag aaacaatgaa ttgattgcag ttggaaaagg cagctcactg aagcggctcc 840
ctgtttttgg aatggagcct cgcatcctat acaacccttt acaaggccag aaatgtattg 900
ttcaaacaac ttcatggtcc cagtgctcaa agacctgtgg aactggtatc tccacacgag 960
ttaccaatga caaccctgag tgccgccttg tgaaagaaac ccggatttgt gaggtgcggc 1020
cttgtggaca gccagtgtac agcagcctga aaaagggcaa gaaatgcagc aagaccaaga 1080
aatcccccga accagtcagg tttacttacg ctggatgttt gagtgtgaag aaataccggc 1140
ccaagtactg cggttcctgc gtggacggcc gatgctgcac gccccagctg accaggactg 1200
tgaagatgcg gttccgctgc gaagatgggg agacattttc caagaacgtc atgatgatcc 1260
agtcctgcaa atgcaactac aactgcccgc atgccaatga agcagcgttt cccttctaca 1320
ggctgttcaa tgacattcac aaatttaggg actaaatgct acctgggttt ccagggcaca 1380
cctagacaaa caagggagaa gagtgtcaga atcagaatca tggagaaaat gggcgggggt 1440
ggtgtgggtg atgggactca ttgtagaaag gaagccttgc tcattcttga ggagcattaa 1500
ggtatttcga aactgccaag ggtgctggtg cggatggaca ctaatgcagc cacgattgga 1560
gaatactttg cttcatagta ttggagcaca tgttactgct tcatttggga gcttgtggag 1620
ttgatgactt tctgttttct gtttgtaaat tatttgctaa gcatattttc tctaggcttt 1680
tttccttttg gggttctaca gtcgtaaaag agataataag attagttgga cagtttaaag 1740
cttttattcg tcctttgaca aaagtaaatg ggagggcatt ccatcccttc ctgaaggggg 1800
acactccatg agtgtctgtg agaggcagct atctgcactc taaactgcaa acagaaatca 1860
ggtgttttaa gactgaatgt tttatttatc aaaatgtagc ttttggggag ggaggggaaa 1920
tgtaatactg gaataatttg taaatgattt taattttata ttcagtgaaa agattttatt 1980
tatggaatta accatttaat aaagaaatat ttacctaata tctgagtgta tgccattcgg 2040
tatttttaga ggtgctccaa agtcattagg aacaacctag ctcacgtact caattattca 2100
aacaggactt attgggatac agcagtgaat taagctatta aaataagata atgattgctt 2160
ttataccttc agtagagaaa agtctttgca tataaagtaa tgtttaaaaa acatgtattg 2220
aacacgacat tgtatgaagc acaataaaga ttctgaagct aaaaaaaaaa

<210> 56
<211> 1636
<212> DNA
<213> Human

<400> 56

cttgaatgaa gctgacacca agaaccgcgg gaagagcttg ggcccaaagc aggaaaggga 60
agcgctcgag ttggaaagga accgctgctg ctggccgaac tcaagcccgg gcgcccccac 120
cagtttgatt ggaagtccag ctgtgaaacc tggagcgtcg ccttctcccc agatggctcc 180
tggtttgctt ggtctcaagg acactgcatc gtcaaactga tcccctggcc gttggaggag 240
cagttcatcc ctaaagggtt tgaagccaaa agccgaagta gcaaaaatga gacgaaaggg 300
cggggcagcc caaaagagaa gacgctggac tgtggtcaga ttgtctgggg gctggccttc 360
agcccgtggc cttccccacc cagcaggaag ctctgggcac gccaccaccc ccaagtgccc 420
gatgtctctt gcctggttct tgctacggga ctcaacgatg ggcagatcaa gatctgggag 480
gtgcagacag ggctcctgct tttgaatctt tccggccacc aagatgtcgt gagagatctg 540
agcttcacac ccagtggcag tttgattttg gtctccgcgt cacgggataa gactcttcgc 600
atctgggacc tgaataaaca cggtaaacag attcaagtgt tatcgggcca cctgcagtgg 660
gtttactgct gttccatctc cccagactgc agcatgctgt gctctgcagc tggagagaag 720
tcggtctttc tatggagcat gaggtcctac acgttaattc ggaagctaga gggccatcaa 780
agcagtgttg tctcttgtga cttctccccc gactctgccc tgcttgtcac ggcttcttac 840
gataccaatg tgattatgtg ggacccctac accggcgaaa ggctgaggtc actccaccac 900
acccaggttg accccgccat ggatgacagt gacgtccaca ttagctcact gagatctgtg 960
tgcttctctc cagaaggctt gtaccttgcc acggtggcag atgacagact cctcaggatc 1020
tgggccctgg aactgaaaac tcccattgca tttgctccta tgaccaatgg gctttgctgc 1080
acattttttc cacatggtgg agtcattgcc acagggacaa gagatggcca cgtccagttc 1140
tggacagctc ctagggtcct gtcctcactg aagcacttat gccggaaagc ccttcgaagt 1200
ttcctaacaa cttaccaagt cctagcactg ccaatcccca agaaaatgaa agagttcctc 1260
acatacagga ctttttaagc aacaccacat cttgtgcttc tttgtagcag ggtaaatcgt 1320
cctgtcaaag ggagttgctg gaataatggg ccaaacatct ggtcttgcat tgaaatagca 1380
tttctttggg attgtgaata gaatgtagca aaaccagatt ccagtgtaca taaaagaatt 1440
tttttgtctt taaatagata caaatgtcta tcaactttaa tcaagttgta acttatattg 1500
aagacaattt gatacataat aaaaaattat gacaatgtcc tgggaaaaaa aaaatgtaga 1560
aagatggtga agggtgggat ggatgaggag cgtggtgacg ggggcctgca gcgggttggg 1620
gaccctgtgc tgcgtt

<210> 57
<211> 460
<212> DNA
<213> Human

<400> 57

ccatgtgtgt atgagagaga gagagattgg gagggagagg gagctcacta gcgcatatgt 60
gcctccaggg ggctgcagat gtgtctgagg gtgagcctgg tgaaagagaa gacaaaagaa 120
tggaatgagc taaagcagcc gcctggggtg ggaggccgag cccatttgta tgcagcaggg 180
ggcaggagcc cagcaaggga gcctccattc ccaggactct ggagggagct gagaccatcc 240
atgcccgcag agccctccct cacactccat cctgtccagc cctaattgtg caggtgggga 300
aactgaggct gggaagtcac atagcaagtg actggcagag ctgggactgg aacccaacca 360
gcctcctaga ccacggttct tcccatcaat ggaatgctag agactccagc caggtgggta 420
ccgagctcga attcgtaatc atggtcatag ctgtttcctg

<210> 58
<211> 1049
<212> DNA
<213> Human

<400> 58

atctgatcaa gaatacctgc cctggtcact ctgcggatgt ttctgtccac ttgttcacat 60
tgaggaccaa gatatccttt tttacagagg cacttgttcg gtctaacaca gacacctcca 120
tgacgacatg ctggctcaca ttttgcagtt ctgcagaagt ccccctccca gcctggacta 180
cagcagcact ttcccgtggg ggtgcagtag ccgtttcgac agagcctgga gcactctgaa 240
gtcagtgtct gtgcaggttg taccgtggct ctgcattcct caggcattaa aggtcttttg 300
ggatctacaa ttttgtagag ttttccattg tgagtctggg tcatactttt actgcttgat 360
aaaatgtaaa cttcacctag ttcatcttct ccaaatccca agatgtgacc ggaaaagtag 420
cctctacagg acccactagt gccgacacag agtggttttt cttgccactg ctttgtcaca 480
ggactttgct ggagagttag gaaattccca ttacgatctc caaacacgta gcttccatac 540
aatctttctg actggcagcc ccggtataca aatccaccaa ccaaaggacc attactgaat 600
ggcttgaatt ctaaaagtga tggctcactt tcataatctt tcccctttat tatctgtaga 660

attctggctg atgatctgtt ttttccattg gagtctgaac acagtatcgt taaattgatg 720
tttatatcag tgggatgtct atccacagca catctgcctg gatcgtggag cccatgagca 780
aacacttcgg ggggctggtt ggtgctgttg aagtgtgggt tgctccttgg tatggaataa 840
ggcacgttgc acatgtctgt gtccacatcc agccgtagca ctgagcctgt gaaatcactt 900
aacccatcca tttcttccat atcatccagt gtaatcatcc catcaccaag aatgatgtac 960
aaaaacccgt cagggccaaa gagcagttgc cctcccagat gctttctgtg gagttctgca 1020
acttcaagaa agactctggc tgttctcaa

<210> 59
<211> 747
<212> DNA
<213> Human

<400> 59

tttttcaaat cacatatggc ttctttgacc ccatcaaata actttattca cacaaacgtc 60
ccttaattta caaagcctca gtcattcata cacattaggg gatccacagt gttcaaggaa 120
cttaaatata atgtatcata ccaacccaag taaaccaagt acaaaaaata ttcatataaa 180
gttgttcaca cgtàggtcct agattaccag cttctgtgca aaaaaaggaa atgaagaaaa 240
atagatttat taactagtat tggaaactaa ctttgtgcct ggcttaaaac ctccctcacg 300
ctcgtctgtc ccacacaaat gtttaagaag tcactgcaat gtactccccg gctctgatga 360
aaagaagccc ctggcacaaa agattccagt gcccctgaag aggctccctt cctcctgtgg 420
gctctcctag aaaaccagcg ggacggcctc cctgctgata ccgtctataa ccttaggggg 480
ccctcgggca ggcaacggca gtggactcat ctcggtgatg gctgtagatg ctaacactgg 540
ccaattcaat gccacaccta ctggttaccc tttgagggca tttctccaga cagaagcccc 600
ttgaagccta ggtagggcag gatcagagat acacccgtgt ttgtctcgaa gggctccaca 660
gcccagtacg acatgcttgc agaagtagta tctctggact tctgcctcca gtcgaccggc 720
cgcgaattta gtagtaatag cggccgc

## Claims

1. Pharmaceutical compositions comprising one or several agents as compound I which modulate the biological function of one or several of the VEGF/VEGF receptor systems, and comprising one or several agents as compound II which modulate the biological function of one or several of the Angiopoietin/Tie receptor systems.

2. Pharmaceutical compositions comprising one or several agents as compound I which are targeted to the endothelium via of one or several of the VEGF/VEGF receptor systems, and comprising one or several agents as compound II which modulate the biological function of one or several of the Angiopoietin/Tie receptor systems.

3. Pharmaceutical compositions comprising one or several agents as compound I which modulates the biological function of one or several of the VEGF/VEGF receptor systems or of one or several of the Angiopoietin/ Tie receptor systems and comprising one or several agents as compound II which are targeted to the endothelium.

4. Pharmaceutical compositions comprising one or several agents as compound I which modulate the biological function of one or several of the VEGF/VEGF receptor systems, and comprising one or several agents as compound II which are targeted to the endothelium via one or several of the Angiopoietin/Tie receptor systems.

5. Pharmaceutical compositions comprising one or several agents as compound I which are targeted to the endothelium via one or several of the VEGF/VEGF receptor systems, and comprising one or several agents as compound II which are targeted to the endothelium via one or several of the Angiopoietin/Tie receptor systems.

6. Pharmaceutical compositions comprising one or several agents as compound I which modulate the biological function of one or several of the VEGF/VEGF receptor systems, and comprising one or several agents as compound II which are targeted to the endothelium via one or several of the VEGF/VEGF receptor systems.

7. Pharmaceutical compositions comprising one or several agents as compound I which modulate the biological function of one or several of the Angiopoietin/Tie receptor systems, and comprising one or several agents as compound II which are targeted to the endothelium via one or several of the Angiopoietin/Tie receptor systems.

8. Pharmaceutical compositions comprising one or several agents which interfere with both the function of one or several of the VEGF/VEGF receptor systems and the function of one or several of the Angiopoietin/Tie receptor systems.

9. Pharmaceutical compositions according to claims 1-8 which are intended for simultaneous or separate sequential therapeutical application.

10. Pharmaceutical compositions according to claims 1-8 which comprise as compound I at least one of
a) compounds which inhibit receptor tyrosine kinase activity,
b) compounds which inhibit ligand binding to receptors,
c) compounds which inhibit activation of intracellular signal pathways of the receptors,
d) compounds which inhibit or activate expression of a ligand or of a receptor of the VEGF or Tie receptor system,
e) delivery systems, such as antibodies, ligands, high-affinity binding oligonucleotides or oligopeptides, or liposomes, which target cytotoxic agents or coagulation-inducing agents to the endothelium via recognition of VEGF/VEGF receptor or Angiopoietin/Tie receptor systems,
f) delivery systems, such as antibodies, ligands, high-affinity binding oligonucleotides or oligopeptides, or liposomes, which are targeted to the endothelium and induce necrosis or apoptosis.

11. Pharmaceutical compositions according to claims 1-8 which comprise as compound II at least one of
g) compounds which inhibit receptor tyrosine kinase activity,
h) compounds which inhibit ligand binding to receptors,
i) compounds which inhibit activation of intracellular signal pathways of the receptors,
j) compounds which inhibit or activate expression of a ligand or of a receptor of the VEGF or Tie receptor system,
k) delivery systems, such as antibodies, ligands, high-affinity binding oligonucleotides or oligopeptides, or liposomes, which target cytotoxic agents or coagulation-inducing agents to the endothelium via recognition of VEGF/VEGF receptor or Angiopoietin/Tie receptor systems,
I) delivery systems, such as antibodies, ligands, high-affinity binding oligonucleotides or oligopeptides, or liposomes, which are targeted to the endothelium and induce necrosis or apoptosis.

12. Pharmaceutical compositions according to claims 1-11 which comprise as compound I and/ or II at least one of Seq. ID Nos. 1-59.

13. Pharmaceutical compositions according to claims 1-11 which comprise as compound I and/ or II Seq. ID Nos. 34a

14. Pharmaceutical compositions according to claims 1-11 which comprise as compound I and/ or II at least one of sTie2, mAB 4301-42-35 and/ or scFv-tTF conjugate.

15. Pharmaceutical compositions according to claims 1-11 which comprise as compound I and/ or II at least one small molecule of genaral formula I 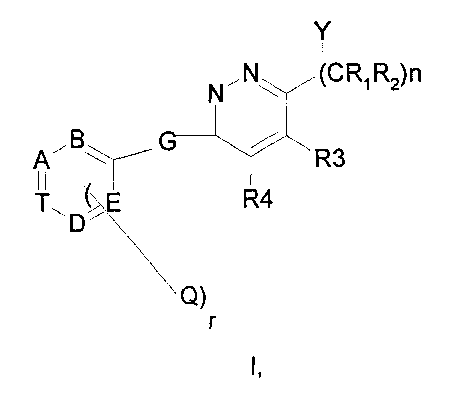 in which
r has the meaning of 0 to 2,
n has the meaning of 0 to 2;
R₃ und R₄
a) each independently from eaxh other have the meaning of lower alkyl,
b) together form a bridge of general partial formula II, 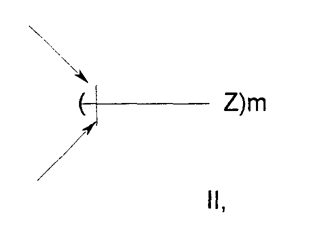
wherein the binding is via the two terminal C- atoms,
and
m has the meaning of 0 to 4; or
c) together form a bridge of partial formula III 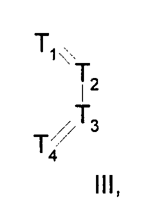 wherein one or two of the ring members T₁,T₂,T₃,T₄ has the meaning of nitrogen, and each others have the meaning of CH, and the bining is via the atoms T₁ and T₄;
G has the meaning of C₁ -C₆ - alkyl, C₂ - C₆ - alkylene or C₂ - C₆ - alkenylene; or C₂ - C₆- alkylene or C₃ -C₆-alkenylene, which are substituted with acyloxy or hydroxy; -CH₂-O-, -CH₂-S-, -CH₂-NH-, -CH₂-O-CH₂-, -CH₂-S-CH₂-, -CH₂-NH-CH₂, oxa (-O-), thia (-S-) or imino (-NH-),
A, B, D, E and T independently from each other have the meaning of N or CH , with the provisio that not more than three of these Substituents have the meaning of N,
Q has the meaning of lower alkyl, lower alkyloxy or halogene,
R₁ and R₂ independently from each other have the meaning of H or lower alkyl,
X has the meaning of imino, oxa or thia;
Y has the meaning of hydrogene, unsubstituted or substituted aryl, heteroaryl, or unsubstituted or substituted cycloalkyl; and
Z has the meaning of amino, mono- or disubstituted amino, halogen, alkyl, substituted alkyl, hydroxy, etherificated or esterificated hydroxy, nitro, cyano, carboxy, esterificated carboxy, alkanoyl, carbamoyl, N-mono- or N, N- disubstituted carbamoyl, amidino, guanidino, mercapto, sulfo, phenylthio, phenyl-lower-alkyl-thio, alkyl-phenyl-thio, phenylsulfinyl, phenyl-lower-alkyl-sulfinyl, alkylphenylsulfinyl, phenylsulfonyl, phenyl-lower-alkan-sulfonyl, or alkylphenylsulfonyl, whereas, if more than one rest Z is present (m≥2), the substituents Z are equal or different from each other, and wherein the bonds marked with an arrow are single or double bonds; or an N-oxide of said compound, wherein one ore more N-atoms carry an oxygene atom, or a salt thereof,
and/or a compound of genaral formula IV 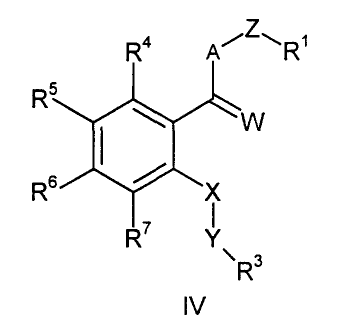 in which
A has the meaning of group =NR²,
W has the meaning of oxygen, sulfur, two hydrogen atoms or the group =NR⁸,
Z has the meaning of the group =NR¹⁰ or =N-, -N(R¹⁰)-(CH₂)_{q}-, branched or unbranched C₁₋₆-Alkyl or is the group 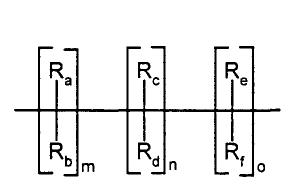 or A, Z and R¹ together form the group 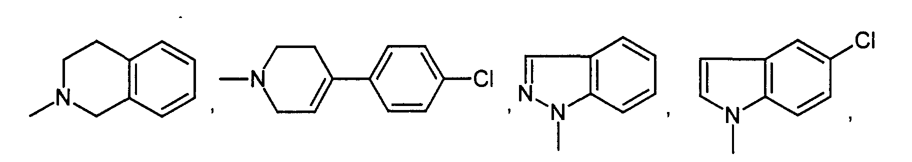 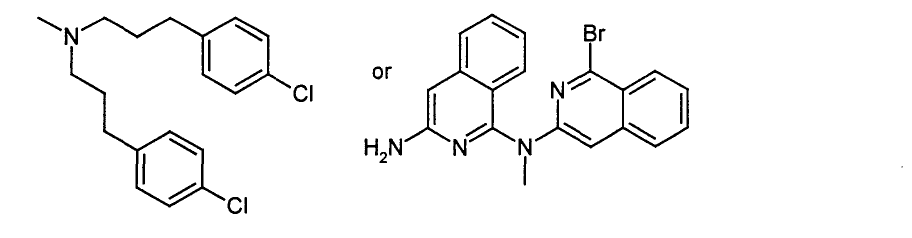
m, n and o has the meaning of 0 - 3,
q has the meaning of 1 - 6,
Rₐ, R_{b}, R_{c}, R_{d}, Rₑ, R_{f} independently from each other have the meaning of hydrogen, C₁₋₄ alkyl or the group =NR¹⁰, and/ or Rₐ and/ or R_{b} together with R_{c} and or R_{d} or R_{c} together with Rₑ and/ or R_{f} form a bound, or up to two of the groups Rₐ-R_{f} form a bridge with each up to 3 C-atoms with R¹ or R²,
X has the meaning of group =NR⁹ or =N-,
Y has the meaning of group -(CH₂)ₚ,
p has the meaning of integer 1-4,
R¹ has the meaning of unsubstituted or optionally substituted with one or more of halogene, C₁₋₆-alkyl, or C₁₋₆-alkyl or C₁₋₆-alkoxy, which is optionally substituted by one or more of halogen, or is unsubstituted or substituted aryl or heteroaryl,
R² has the meaning of hydrogen or C₁₋₆-alkyl, or form a bridge with up to 3 ring atoms with Rₐ-R_{f} together with Z or R₁,
R³ has the meaning of monocyclic or bicyclic aryl or heteroaryl which is unsubstituted or optionally substituted with one or more of für halogen, C₁₋₆-alkyl, C₁₋₆-alkoxy or hydroxy,
R⁴ ,R⁵, R⁶ and R⁷ independently from each other have the meaning of hydrogen, halogene or C₁₋₆-alkoxy, C₁₋₆-alkyl or C₁₋₆-carboxyalkyl, which are unsubstituted or optionally substituted with one or more of halogene, or R⁵ and R⁶ together form the group 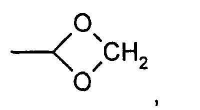
R⁸, R⁹ and R¹⁰ independently from each other have the meaning of hydrogen or C₁₋₆-alkyl, as well as their isomers and salts,
and/ or a compound of general formula V 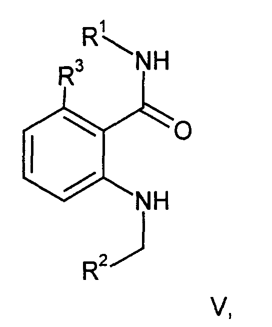 in which
R¹ has the meaning of group 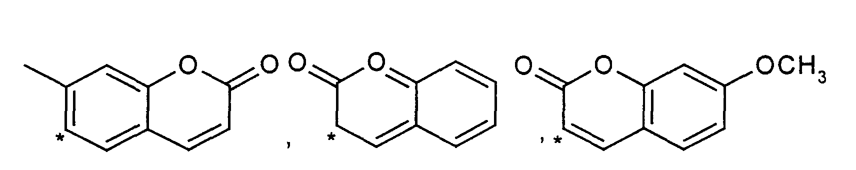 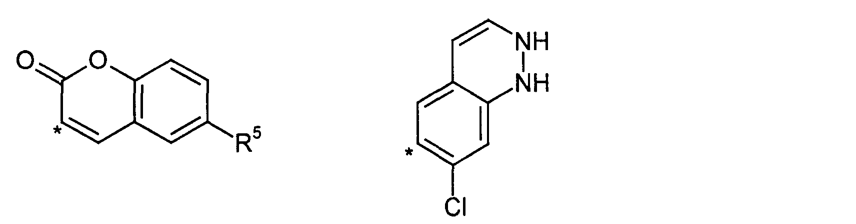 in which R⁵ is chloro, bromo or the group -OCH₃, 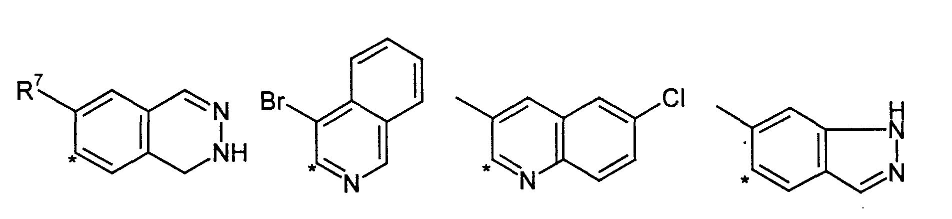 in which R⁷ is -CH₃ or chloro, 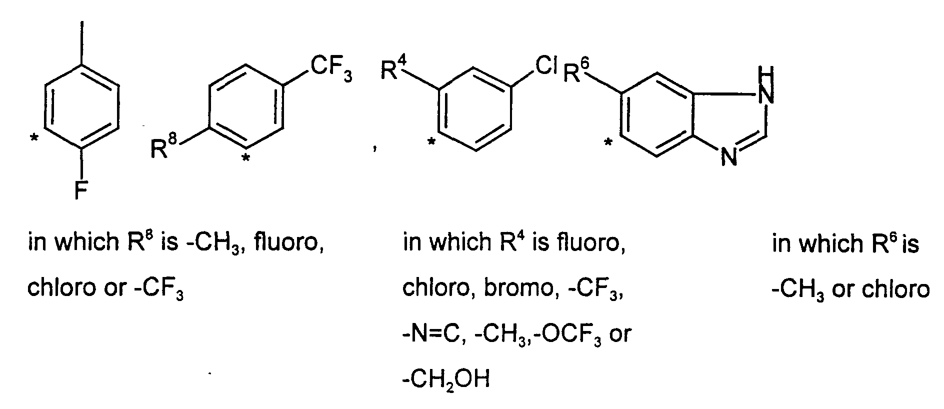
R² has the meaning of pyridyl or the group 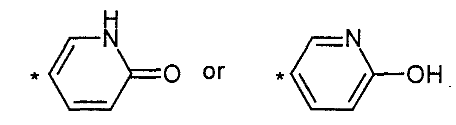 and
R³ has the meaning of hydrogen or fluoro, as well as their isomers and salts.

16. Pharmaceutical compositions according to claim 15 which comprise as compound I and/ or II (4-Chlorophenyl)[4-(4-pyridylmethyl)-phthalazin-1-yl]ammonium hydrogen succinate

17. Pharmaceutical compositions according to claims 1-16 which comprise as compound I (4-Chlorophenyl)[4-(4-pyridylmethyl)-phthalazin-1-yl]ammonium hydrogen succinate, sTie2, mAB 4301-42-35 and/ or scFv-tTF conjugate, and as compound II (4-Chlorophenyl)[4-(4-pyridylmethyl)-phthalazin-1-yl]ammonium hydrogen succinatesTie2, mAB 4301-42-35 and/ or scFv-tTF conjugate, with the provisio that compound I is not identically to compound II.

18. Pharmaceutical compositions according to claims 1-17 which comprise as compound I (4-Chlorophenyl)[4-(4-pyridylmethyl)-phthalazin-1-yl]ammonium hydrogen succinate and as compound II sTie2, mAB 4301-42-35 and/ or scFv-tTF conjugate.

19. Pharmaceutical compositions according to claims 1-17 which comprise as compound I mAB 4301-42-35 and as compound II sTie2, and/ or scFv-tTF conjugate.

20. Pharmaceutical compositions according to claims 1-17 which comprise as compound I scFv-tTF conjugate and as compound II sTie2 and/ or mAB 4301-42-35.

21. Use of pharmaceutical compositions according to claims 1-20, for the production of a medicament for the treatment of tumors, cancers, psoriasis, arthritis, such as rheumatoide arthritis, hemangioma, angiofribroma, eye diseases, such as diabetic retinopathy, neovascular glaukoma, kidney diseases, such as glomerulonephritis, diabetic nephropathie, maligneous nephrosclerosis, thrombic microangiopatic syndrome, transplantation rejections and glomerulopathy, fibrotic diseases, such as cirrhotic liver, mesangial cell proliferative diseases, artheriosclerosis, damage of nerve tissues, suppression of the ascites formation in patients and suppression of VEGF oedemas.
